(19) Europäisches Patentamt
European Patent Office
Office européen des brevets

(11) Numéro de publication : **0 210 896 B2**

(12) # NOUVEAU FASCICULE DE BREVET EUROPEEN

(45) Date de publication du nouveau fascicule du brevet : **16.03.94 Bulletin 94/11**

(21) Numéro de dépôt : **86401473.3**

(22) Date de dépôt : **03.07.86**

(51) Int. Cl.$^5$ : **C07C 227/22,** C07C 229/06, C07C 271/24, C07D 207/273, C07D 207/38

(54) **Dérivés d'acides amino-4 hydroxy-3 carboxyliques optiquement purs et procédé de synthèse stéréospécifique.**

(30) Priorité : **03.07.85 FR 8510181**

(43) Date de publication de la demande : **04.02.87 Bulletin 87/06**

(45) Mention de la délivrance du brevet : **10.10.90 Bulletin 90/41**

(45) Mention de la décision concernant l'opposition : **16.03.94 Bulletin 94/11**

(84) Etats contractants désignés : **AT BE CH DE FR GB IT LI LU NL SE**

(56) Documents cités :
**EP-A- 155 809**
**WO-A-92/13545**
**US-A- 4 397 786**
**US-A- 4 477 440**
**HELVETICA CHIMICA ACTA, vol. 60, no. 71, fasc. 2, 1977, pages 660-669, Basel, CH; W. HOFHEINZ et al.: "Dysidin, ein neuartiger, chlorhaltiger Naturstoff aus dem Schwamm Dysidea herbacea"**
**JOURNAL OF MEDICINAL CHEMISTRY, vol. 8, juillet 1965, pages 478-482, Washington, D.C., US; S.A. HARRIS et al.: "The synthesis of tenuazonic and congeneric tetramic acids"**
**CHEMICAL AND PHARMACEUTICAL BULLETIN, vol. 32, no. 8, 1984, pages 3291-3298; KUNIYOSHI TANAKA et al.: "Syntheses and antimicrobial activities of five-membered heterocycles having a phenylazo substituent"**
**JOURNAL OF ORGANIC CHEMISTRY, vol. 47, no. 8, 1982, pages 1534-1546, American Chemical Society, Washington, D.C., US; E. VEDEJS et al.: "Synthesis of the cytochalasin D isoindolone unit: Solutions to the problem of regiochemistry in N-benzoylpyrrolinone diels-alder reactions"**

(73) Titulaire : **ELF SANOFI**
**32-34, rue Marbeuf**
**F-75008 Paris (FR)**
Titulaire : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **Jouin, Patrick**
**9, rue du Mazel**
**F-34150 Amiane (FR)**
Inventeur : **Nisato, Dino**
**4, Impasse de l'Olivette**
**F-34680 Saint-Georges-d'Orques (FR)**
Inventeur : **Castro, Bertrand**
**L'Estaple Avenue des Costières**
**F-34130 Saint-Aunes (FR)**

(74) Mandataire : **Gillard, Marie-Louise et al**
**Cabinet Beau de Loménie 158, rue de l'Université**
**F-75340 Paris Cédex 07 (FR)**

EP 0 210 896 B2

Jouve, 18, rue Saint-Denis, 75001 PARIS

(56) Documents cités :

CHEMICAL ABSTRACTS, vol. 99, no. 9, 29 août 1983, page 667, no. 71139g, Columbus, Ohio, US; J. GUMIENIAK et al.: "Syntheses of edeine antibiotics and their analogs. Part V. Absolute configuration of 2,6-diamino-7-hydroxyazelaic acid isolated from edeines", & POL. J. CHEM. 1981, 55(7-8), 1519-26; & CHEMICAL ABSTRACTS, American Chemical Society, vol. 99, Chemical Substance Index, O-P, (partie 4 des 5 parties) juillet-décembre 1983, Chemical Abstracts Service, page 6065CS

TETRAHEDRON, vol. 34, 1978, pages 223-231, Pergamon Press, Oxford, GB; J.L. VAN DER BAAN et al.: "The total synthesis of the antibiotic malonomicin (K16)"

THE JOURNAL OF ANTIBIOTICS, vol. 33, no. 2, février 1980, pages 173-181; SOICHIRO TODA et al.: "Bu-2313, A new antibiotic complex active against anaerobes III. Semi-synthesis of Bu-2313 A and B, and their analogs"

THE JOURNAL OF ANTIBIOTICS, vol. 30, no. 10, octobre 1977, pages 789-805; MASATAKA KONISHI et al.: "Tallysomycin, a new antitumor antibiotic complex related to bleomycin II. Structure determination of tallysomycins A and B"

JOURNAL OF THE CHEMICAL SOCIETY. CHEMICAL COMMUNICATIONS, 1974, pages 419,420, Londres, GB; J.R. HLUBUCEK et al.: "A key intermediate for the synthesis of some nuclear analogues of the penicillins and cephalosporins"

JOURNAL OF THE CHEMICAL SOCIETY PERKIN TRANSACTIONS 1., 1972, pages 2121-2128, Londres, GB; T.P.C. MULHOLLAND et al.: "Synthesis of pyrrolidine-2,4-diones (tetramic acids) and some derivatives"

BULLETIN OF THE CHEMICAL SOCIETY OF JAPAN, vol. 49, no. 11, novembre 1976, pages 3287-3290, Tokyo, JP; T. KATSUKI et al.: "The stereoselective synthesis of threo-3-hydroxy-4-amino acids"

THE JOURNAL OF ANTIBIOTICS, vol. 37, no. 12, décembre 1984, pages 1546-1552; SHIGETOSHI TSUBOTANI et al.: "Cephabacins, new cephem antibiotics of bacterial origin III. Structural determination"

CHEMICAL ABSTRACTS, vol. 99, no. 1, 4 juillet 1983, page 549, no. 5983b, Columbus, Ohio, US; H. WOJCIECHOWSKA et al.: "The selectivity of amino acids and peptides amidination with O-methylisourea" & ACTA BIOCHIM. POL. 1982, 29(3-4), 197-204; & CHEMICAL ABSTRACTS, American Chemical Society, vol. 99, Chemical Substance Index, C-F, (partie 2 des 5 parties) juillet-décembre 1983, Chemical Abstracts Service, page 2309CS

CHEMICAL ABSTRACTS, vol. 103, no. 15, 14 octobre 1985, page 768, no. 123872p, Columbus, Ohio, US; J. GUMIENIAK et al.: "Syntheses of edeine antibiotics and their analogs. Part VI. Synthesis of protected (3R,4S)- and (3S,4S)-4,8-diamino-3-hydroxyoctanoic acid" & POL. J. CHEM. 1984, 58(7-8), 881-5

PROSTAGLANDINS, vol. 13, no. 4, avril 1977, pages 611-618; SALVADOR MONCADA et al.: "IMIDAZOLE: A selective inhibitor of thromboxane synthetase"

(56) Documents cités :

D.H. RICH et al., J. Med. Chem., 1980, (23), pages 27-33

SZEWCZUK, Z. et al., Int. J. Peptide Protein Res. 40, 1992, pages 233-242

MAIBAUM, J. et al., J. Med Chem.., 1989, 32, pages 1571-1576

JOUIN, P. et al., J. Chem. Soc. Perkins Trans. I 1987, pages 1177-1182

## Description

La présente invention a pour objet un procédé de synthèse de dérivés d'acides amino-4 hydroxy-3 carboxyliques optiquement purs, chaque étape de ce procédé étant réalisée de façon stéréocontrôlée.

Par un autre de ses aspects, la présente invention concernne les nouveaux dérivés d'acides amino-4 hydroxy-3 carboxyliques optiquement purs obtenus par ce procédé.

Le procédé selon l'invention permet de préparer la statine et ses dérivés. Ces composés sont utiles notamment dans la préparation de peptides inhibiteurs de la rénine.

La présente invention se réfère également aux nouveaux dérivés optiquement actifs de la pyrrolidinedione et de la pyrrolidinone obtenus comme intermédiaires de synthèse du procédé selon l'invention.

Dans la description de la présente invention et dans les revendications, les abréviations suivantes seront utilisées :

Leu : L-leucine
Phe : L-phénylalanine
Sta : Statine = Acide (3S,4S) amino-4 hydroxy-3 méthyl-6 heptanoïque
AcOH : Acide acétique
AcOEt : Acétate d'éthyle
Boc : tert-butyloxycarbonyle
Z : benzyloxycarbonyle
CCM : chromatographie en couche mince
DMSO : diméthylsulfoxyde
MeOH : méthanol
Acide de Meldrum : diméthyl-2,2 dioxanne-1,3 dione-4,6.
Les carbones asymétriques sont indiqués C.

Plusieurs méthodes de synthèse de dérivés d'acides amino-4 hydroxy-3 carboxyliques ont été décrites, notamment la préparation de l'acide (3S,4S)-amino-4 hydroxy-3 méthyl-6 heptanoïque : la statine.

Aucune de ces méthodes n'est entièrement stéréospécifique et la séparation des diastéréoisomères constitue toujours une étape nécessaire. Jusqu'à ce jour, les nouvelles voies de synthèse décrites ont surtout tenté d'améliorer les résultats obtenus dans cette étape (Bull. Soc. Chim. Fr., 1983, 230-232). Malgré son titre : "The Stereoselective Synthesis of threo-3-hydroxy-4-amino Acids", l'article publié dans Bull. Chem. Soc. Jap., 1976, 49 (11), 3287-3290, ne résout pas le problème de la stéréospécificité de la synthèse. Les auteurs décrivent la préparation de certains acides amino-4 hydroxy-3 carboxyliques, la statine par exemple, à partir de l'aminoacide correspondant en opérant selon la schéma réactionnel simplifié suivant :

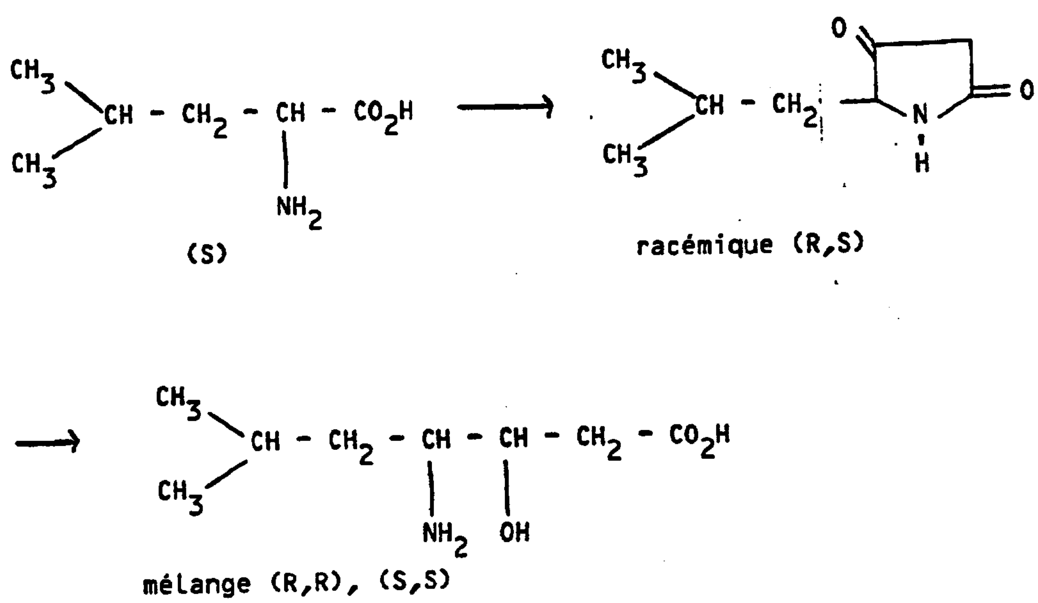

(S) racémique (R,S)

mélange (R,R), (S,S)

la pyrrolidinedione intermédiaire est racémique, elle subit ensuite une réduction stéréosélective. La réduction conduit au mélange des diastéréoisomères (R,R et S,S) de configuration cis.

La présente invention a pour objet un procédé de préparation stéréospécifique de dérivés d'acides (3R,4R)- ou, respectivement, (3S,4S)-amino-4 hydroxy-3 carboxyliques optiquement purs de formule:

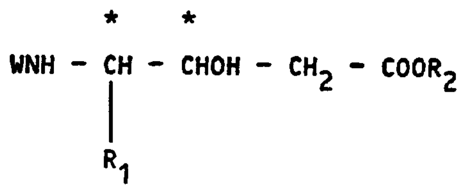 (I)

dans laquelle :

- W est l'hydrogène ou un groupe N-protecteur ;
- $R_1$ représente un radical alkyle, linéaire ou ramifié ayant de 1 à 6 atomes de carbone tels que méthyle, éthyle, n-propyle, isopropyle, n-butyle, isobutyle, tertiobutyle, méthyl-1 propyle, n-pentyle ou n-hexyle, un radical méthyloxyalkyle inférieur tel que méthyloxyéthyle, un radical benzyloxyalkyle inférieur, un radical méthylthioalkyle inférieur tel que méthylthiométhyle, méthylthioéthyle, un radical phénylthioalkyle inférieur, un radical benzylthioalkyle inférieur, un radical aminoalkyle inférieur libre ou substitué par un groupe protecteur sur la fonction amine tel que amino-4 butyle ou benzyloxycarbonylamino-4-butyle, un radical (alkyle inférieur)-aminoalkyle inférieur tel que méthylamino-, éthylamino-, isopropylamino- ou tertiobutylamino-éthyle, un radical (dialkyle inférieur)-aminoalkyle inférieur tel que diméthylamino-, diéthylamino-, di n-propylamino- ou di n-butylaminoéthyle, un radical hydroxyalkyle inférieur tel qu'hydroxyméthyle, ou hydroxy-1 éthyle, un radical carboxyalkyle inférieur libre ou estérifié tel que carboxyméthyle ou carboxyéthyle, ou un radical carboxamidoalkyle inférieur libre ou alkylé, un radical alkyle inférieur substitué à la fois et sur le même atome de carbone par une amine et un carboxy libre ou estérifié, un radical alcényle, linéaire ou ramifié, ayant de 2 à 6 atomes de carbone, un radical méthyloxyalcényle inférieur tel que méthyloxyvinyle, un radical phénoxyalcényle inférieur, un radical benzyloxyalcényle, un radical méthylthioalcényle inférieur tel que méthylthiovinyle, un radical phénylthioalcényle inférieur, un radical benzylthialcényle inférieur, un radical aminoalcényle inférieur libre ou protegé sur la fonction amine tel que aminoallyle, un radical (alkyle inférieur)-aminoalcényle inférieur tel que méthylamino-, éthylamino-, isopropylamino- ou tertiobutylamino-allyle, un radical (dialkyle inférieur)-aminoalcényle inférieur tel que diméthylamino-, diéthylamino, di n-propylamino-, di n-butylamino-allyle, un radical carboxylalcényle inférieur libre ou estérifié, un radical carboxamidoalcényle inférieur libre ou alkylé, un radical alcynyle linéaire ou ramifié ayant de 2 à 6 atomes de carbone,
  ou $R_1$ représente l'un des radicaux de formules générales :

  Cy-A-, Cy-O-A'- ou $R_3$-A'-

  dans laquelle :
  . Cy représente un radical hydrocarboné aromatique ou alicyclique ou un radical hétérocyclique contenant un atome d'oxygène ou de soufre ou un ou deux atomes d'azote, Cy étant éventuellement mono-, di- ou tri-substitué par des radicaux hydroxy, alkyle inférieur, alcoxy inférieur, trifluorométhyle, nitro ou halogéno,
  . A représente une liaison simple ou un radical alkylène, linéaire ou ramifié, ayant de 1 à 5 atomes de carbone ou un radical alcénylène, linéaire ou ramifié, ayant de 2 à 5 atomes de carbone.
  . A' représente un radical alkylène, linéaire ou ramifié, ayant de 1 à 5 atomes de carbone ou un radical alcénylène, linéaire ou ramifié, ayant de 2 à 5 atomes de carbone.
  . $R_3$ représente un groupement S- protecteur ;
- $R_1$ représentant de préférence un radical alkyle linéaire ou ramifié de 1 à 6 atomes de carbone, un benzyle, un cyclohexylméthyle, un méthylthioalkyle inférieur, un benzyloxyalkyle inférieur, un aminoalkyle inférieur libre ou substitué sur la fonction alkyle par un groupe N-protecteur, un indolyl-3 méthyle.
- $R_2$ représente l'hydrogène, un métal alcalin ou alcalino-terreux, un alkyle inférieur, un benzyle non substitué ou substitué par un groupement alkyle inférieur, par un halogène ou par un groupement nitro.

Dans le présente contexte, les terms repris ci-dessus comportent la signfication suivante :

- "alkyle inférieur" désigne les restes d'hydrocarbures aliphatiques saturés contenant jusqu'à 4 atomes de carbone tels que méthyle, éthyle, n-propyle, isopropyle, n-butyle, isobutyle ou tertiobutyle ;
- "alcoxy inférieur" désigne le groupement hydroxy substitué par un radical alkyl inférieur tel que défini ci-dessus ;
- "radical hydrocarboné aromatique ou alicyclique" signifie un radical phényle, naphtyle, cycloalkyle de 3 à 7 atomes de carbone tels que cyclopropyle, cyclobutyle, cyclopentyle, cyclohexyle ou cycloheptyle ;
- "radical hétérocyclique" signifie un radical furyle, benzofuryle, thiényle, benozthiényle, pyrrolyle, pyridyle, pipéridyle, morpholyle, pipérazinyle, N-(alkyle inférieur)-pipérazinyle, indolyle ou 1H-imidazolyle;
- "groupement S- protecteur" désigne un groupement protecteur facilement éliminable pour groupements mercapto tels que par exemple, un groupement aralkyle tel que benzyle, un groupement aralkyle substitué tel que p-nitrobenzyle ;

- par "groupe N-protecteur", on entend un groupe protecteur utilisé normalement dans la chimie des peptides tel que, à titre d'exemple, un groupement alkylcarbonyle tel que formyle, acétyle ou propionyle, un groupement alcoxycarbonyle tel que tert-butyloxycarbonyle, un groupement alcoxyalkylcarbonyle tel que méthoxyacétyle ou méthoxypropionyle, un groupement alcoxycarbonyle tel que méthoxyacétyle ou méthoxycabonyle, un groupement aralkyloxycarbonyle tel que benzyloxycarbonyle, un groupement aralkyloxycarbonyle substitué tel que p-nitrobenzyloxycarbonyle, un groupement trityle ou méthoxytrityle ou un groupement tert-butyloxycarbonyle (Boc) ou benzyloxycarbonyle (Z) constituant les groupes N-protecteurs préférés.

Le procédé de préparation stéréospécifique des dérivés d'acides amino-4 hydroxy-3 carboxyliques de formule (I) est caractérisé en ce que l'on fait réagir l'acide de Meldrum sur un amino acide optiquement pur, de configuration D ou, respectivement, L, protégé de formule :

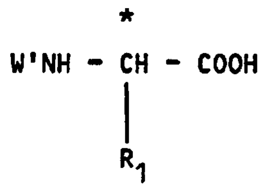
(II)

dans laquelle W' représente un groupe N-protecteur tel que Boc ou Z et $R_1$ a la signification indiquée plus haut, en milieu basique, en présence d'un agent activateur de l'acide aminé (II) ou d'un agent de couplage pour obtenir le composé (III) ; par chauffage dans un solvant entre 30°C et 100°C on obtient le composé (IV-IVa) sous 2 formes tautomères en équilibre dans lesquelles la chiralité de l'atome de carbone portant le substituant $R_1$ est conservée

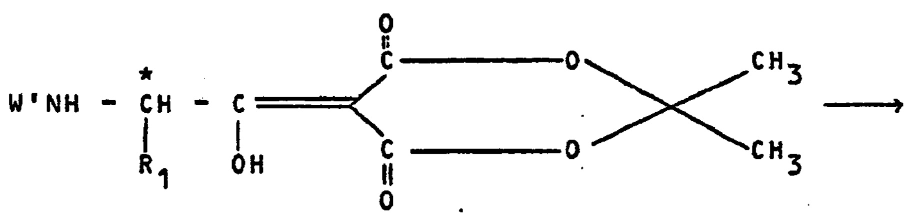
(III)

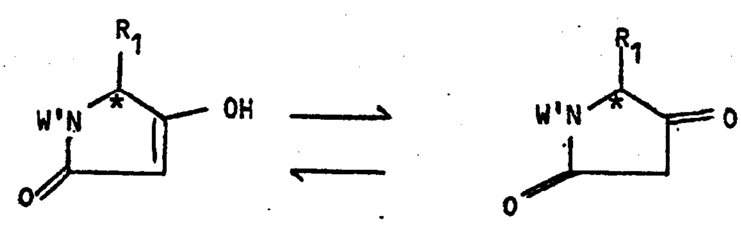
(IV) (IVa) ;

on réduit (IV-IVa) par un borohydrure métallique en milieu acide pour obtenir (V) de configuration Cis

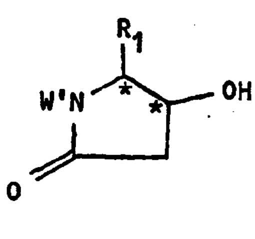
(V) ;

on ouvre ensuite le cycle pour obtenir (I) dans lequel W = W' soit en milieu acide lorsque W' représente un

groupe N-protecteur stable en milieu acide tel que Z, soit en milieu basique lorsque W' représente un groupe N-protecteur stériquement encombré et labile en milieu acide tel que Boc ; pour obtenir le composé (I) dans lequel W = H, on effectue un déprotection de l'azote selon les méthodes connues.

Par un autre de ses aspects, la présente invention se réfère aux produits nouveaux, dérivés d'acide (3R,4R)- ou, respectivement, (3S,4S)-amino-4 hydroxy-3 carboxyliques, obtenus par le procédé selon l'invention, répondant à la formule :

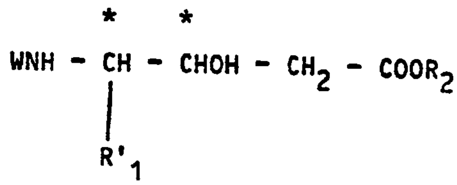

(VII)

dans laquelle W et $R_2$ ont les mêmes significations que précédemment et $R'_1$ a la même signification que $R_1$ à la condition que $R'_1$ soit autre que :

- alkyle en $C_1$-$C_6$ ;
- Cy-A- dans lequel Cy est un radical hydrocarboné aromatique ou alicyclique, et A est un alkylène en $C_1$-$C_5$ ;
- benzyle substitué par un nitro, oméga aminobutyle non substitué ou substitué par un groupe protecteur sur l'amine ;
- oméga amino-oméga carboxybutyle.

Les composés (VII) sont des dérivés de la statine ; ils peuvent comme la statine être utilisés dans la synthèse de nouveaux dérivés peptidiques inhibiteurs de la rénine.

La présente invention se réfère également aux hydroxy-4 1, 2-dihyropyrrolone-2 substituées en 5, optiquement pures, nouvelles, obtenues comme intermédiaires de synthèse du procédé selon l'invention, répondant à la formule :

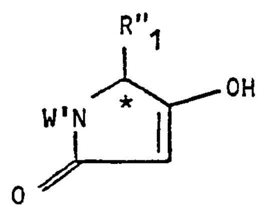

(IV)

qui existent également sous forme tautomère de pyrrolidinedione-3,5 substituées en 2:

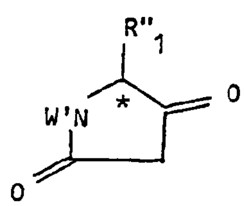

(IVa)

dans lesquelles W' a la même signification que précédemment et $R''_1$ a la même signification que $R_1$ avec la limitation que $R'_1$ est autre que amino-4 butyle ou amino-4 carboxy-4 butyle.

Enfin la présente invention se réfère aux pyrrolidinones optiquement pures, de configuration cis, nouvelles, obtenues comme intermédiaires de synthèse du procédé selon l'invention de formule:

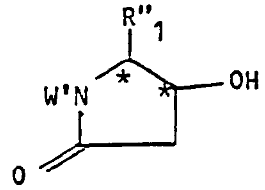

(V)

dans laquelle W' et R"$_1$ ont les significations indiquées ci-dessus.

Les composés utiles comme intermédiaires dans le procédé de la présente invention et illustrés par les formules IV, IVa et V ci-dessus peuvent être regroupés dans la formule suivante

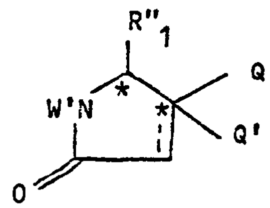

(VI)

dans laquelle W' et R"$_1$ sont tels que définis ci-dessus, Q représente un groupe hydroxyle, Q' n'existe pas ou représente un atome d'hydrogène, ou bien Q et Q', pris ensemble, représentent un atome d'oxygène et la ligne en pointillé représente, lorsque Q' n'existe pas, une liaison supplémentaire, étant entendu que, lorsque Q' représente un atome d'hydrogène, le composé VI a la configuration (S, S) ou (R, R).

On notera que les composés ci-dessus de formule VI dans laquelle W' est l'hydrogène et R" est le groupe méthyle, isopropyle, isobutyle, sec-butyle ou benzyle ont été décrits dans l'art antérieur cité ci-après:

- Bull. Chem. Soc. Japon 1976, 49 (11), 3287-3290
- Helvetica Chimica Acta, 1977,60(71), 660-669)
- J. Med. Chem. 1965, 8, 478-482
- Chem. Pharm. Bull., 1984, 32 (8), 3291-3298
- J. Org. Chem. 1982, 47 1534-1546.

Le procédé selon l'invention apporte un avantage tout à fait nouveau notamment grâce à la stéréospécificité de toutes ses étapes qui permet d'obtenir chacun des composés intermédiaires sous une forme optiquement pure et d'éviter ainsi la séparation d'isomères optiques. La chiralité de l'atome de carbone en alpha de l'acide aminé de départ est conservée tout au long du procédé. Ainsi, après la condensation avec l'acide de Meldrum, on obtient par chauffage la pyrrolidinone (IV-IVa) optiquement pure protégée dont la réduction puis l'ouverture sont ensuite réalisées de façon stéréospécifique dans les conditions opératoires selon l'invention.

Le produit de départ est un alpha aminoacide optiquement pur, protégé, de formule :

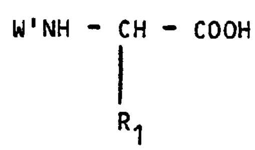

(II)

dans laquelle: W' représente un groupement protecteur tel que Boc ou Z et R$_1$ a la signification indiquée plus haut.

On fait réagir l'acide de Meldrum sur l'acide aminé (II), activé par un agent activateur tel qu'un chlorure d'acide ou un chloroformiate insaturé, ou sur l'acide aminé (II) en présence d'un agent de couplage tel que la dicyclohexylcabodiimide. La réction est effectuée dans un milieu rendu basique par addition d'une base telle que la pyridine, ou la diméthylamino-4 pyridine, à une tempérautre comprise entre la température ambiante et -10°C, dans un solvant aprotique tel que le chlorure de méthylène ou l'acétate d'éthyle, pendant plusieurs heures (de 1 à 5 h).

(II) + 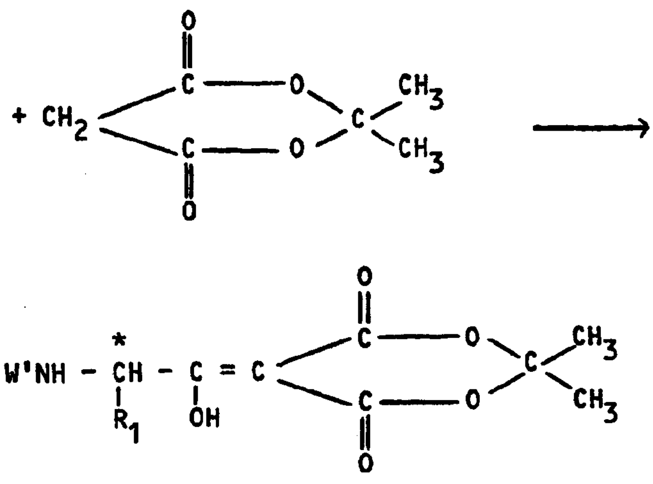

(III)

On traite le milieu réactionnel selon les méthodes habituelles de la chimie organique par addition d'une solution aqueuse faiblement acide, d'une solution saline, puis séchage de la phase organique et évaporation sous pression réduite. Le compose (III) ainsi obtenu est repris dans un solvant et chauffé à une température comprise entre 30°C et 100°C. On traite le milieu réactionnel selon les méthodes habituelles de la chimie organique pour extraire et purifier le composé (IV-IVa) de formule :

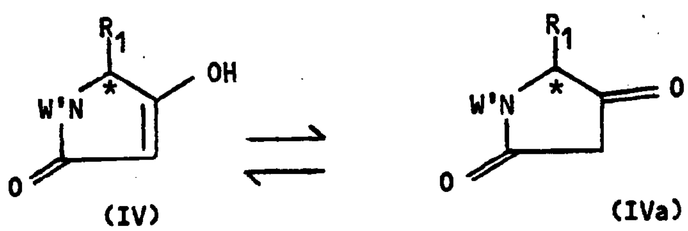

(IV) (IVa)

La réaction (III)→(IV-IVa) s'effectue de façon stéréocontrôlée. Cela est vérifié par la mesure du pouvoir rotatoire de (IV-IVa). On effectue ensuite la réduction de (IV-IVa) par un borohydrure métallique en milieu acide tel que, par exemple, le borohydrure de sodium en présence d'acide acétique dans un solvant aprotique, tel que le chlorure de méthylène. Lorsque le substituant $R_1$ du composé (IV-IVa) n'est pas sensible à l'hydrogénation, on peut également effectuer une réduction catalytique sous pression d'hydrogène, par exemple, en présence d'oxyde de platine ou de nickel de Raney. Le produit réduit est purifié par une technique classique telle que la chromatographie sur colonne pour obtenir le composé (V):

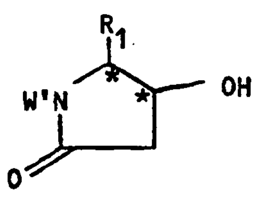

(V)

La réduction est stéréospécifique et conduit au composé de configuration cis par induction asymétrique. On réalise enfin l'ouverture de la pyrrolidinone (V).

Lorsque le groupe protecteur est stable en milieu acide, comme par exemple Z, l'ouverture de (V) est effectuée en milieu acide soit dans un solvant organique tel que le dioxanne pour obtenir (I) sous forme acide ($R_2$ = H), soit dans une solution alcoolique $R_2OH$ pour obtenir (I) sous forme d'un ester ($R_2$ est tel que défini ci-dessus et différent de H).

Lorsque le groupe protecteur W' est stériquement encombré et qu'il est labile en milieu acide, comme par exemple Boc, l'ouverture est effectuée en milieu basique, à savoir soit en présence de soude pour obtenir (I)

sous forme acide ($R_2$ = H), soit dans une solution alcoolique $R_2OH$ en présence de l'alcoolate correspondant pour obtenir (I) sous forme ester ($R_2$ est tel que défini ci-dessus et différent de H).

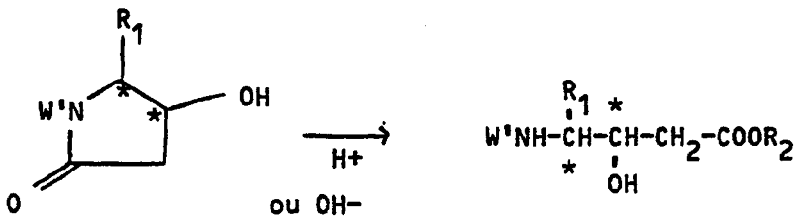


(V) (I')

La chiralité de l'atome de carbone portant l'alcool secondaire est conservée (Bull. Chem. Soc. Jap., 1976, 49 (II), 3287-3290). On peut ensuite effectuer une déprotection sur l'azote selon les méthodes connues de l'homme de l'art.

Il est évident que, lorsque l'on part d'un acide aminé (II) de configuration L, on obtient sélectivement le composé (I) de configuration (3S,4S) alors que, respectivement, si l'on part de l'acide aminé (II) de configuration D, on obtient sélectivement le composé (I) de configuration (3R,4R).

Les exemples suivants non limitatifs sont donnés à titre d'illustration de la présente invention.

Les points de fustion (Fc) sont mesurés au tube capillaire.

Les indices de rotation (alpha D) sont mesurés à 25°C, le produit ayant la concentration molaire dans le méthanol, sauf indication contraire.

Les spectres de résonance magnétique nucléaire du proton (RMN) sont enregistrés à 360 MHz en solution dans le DMSO, l'étalon interne étant l'hexaméthyldisiloxanne. Lorsque le spectre RMN du carbone 13 est enregistré c'est également à 360 MHz, en solution dans le DMSO, l'étalon interne étant l'hexaméthyldisiloxanne.

Les abréviations suivantes sont utilisées :

- s : singulet
- d : doublet
- dd : doublet de doublet
- m : multiplet
- t : triplet
- q : quadruplet
- qd : quadruplet dédoublé.

Les déplacements chimiques (delta) sont mesurés en ppm.

EXEMPLE 1 :

Acide (3S,4S) tert-butyloxycarbonylamino-4 hydroxy-3 méthyl-6 heptanoïque.

1) Boc Leucine déshydratée :

Dans un ballon rodé de 500 ml, on mélange 5 g de Boc-Leu-Oh, $H_2O$ et 100 ml de toluène, puis on effectue 2 fois de suite une évaporation azéotropique de l'eau à l'évaporateur rotatif. Le produit déshydraté, sous forme d'une huile, est laissé une nuit au dessiccateur sur anhydride phosphorique.

2) ((tert-butyloxycarbonylamino-2 hydroxy-1 méthyl-5) pentylidène)-5 diméthyl-2,2 dioxanne-1,3 dione-4,6.

Composé (III) : W' = Boc, $R_1 = CH_2CH(CH_3)_2$.

La Boc Leucine déshydratée obtenue précédemment est reprise dans 100 ml de chlorure de méthylène auxquels on ajoute 3 g d'acide de Meldrum, puis le milieu est mis sous agiatation à -5°C et on ajoute 5,6 g de diméthylamino-4 pyridine puis goutte à goutte en 30 min 2,6 ml de chlroformiate d'isopropényle dilués dans 10 ml de chlorure de méthylène. Après 2 h, le milieu réactionnel est lavé par 200 ml d'une solution de sulfate acide de potassium à 5 %, puis à l'eau, séché sur sulfate de sodium et évaporé sous pression réduite à une température inférieure à 40°C. La réaction est suivie en CCM en éluant par le mélange AcOEt/MeOH/AcOH : 95/3/2 ; Rf = 0,60.

3) (S) tert-butyloxycarbonyl-1 hydroxy-4 isobutyl-5 1,2-dihydropyrrolone-2.

Composé (IV-IVa) : W' = Boc, $R_1 = CH_2CH(CH_3)_2$.

Le produit brut obtenu précédemment est repris par 100 ml d'acétate d'éthyle. La solution est chauffée à reflux pendant 20 min puis le solvant est évaporé. La réaction est suivie en CCM ; le produit est extrait du milieu réactionnel par une solution de bicarbonate de sodium à 5 %, on acidifie par de l'acide citrique

en poudre, puis on extrait par de l'acétate d'éthyle. La phase organique est séchée sur sulfate de sodium et évaporée. On obtient 4,5 g de produit.

Rendement : 88 %.

Rf : (AcOEt/MeOH/AcOH : 95/3/2) = 0,38 ;

alpha D = +101.

## SPECTRE DE RMN

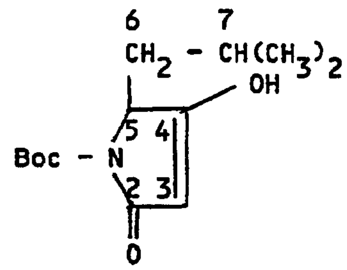

| Delta | Aspect | Intégration | Attribution |
|---|---|---|---|
| 1,40 | s | 9 H | $C(CH_3)_3$ |
| 4,85 | s | 1 H | CH en 3 |
| 4,33 | q | 1 H | CH en 5 |
| 13 | s | 1 H | OH |
| 1,70 | m | 2 H | $CH_2$ en 6 |
| 1,60 | m | 1 H | CH en 7 |
| 0,79 | q | 6 H | $(CH_3)_2$ |

4) (S,S) (tert-butyloxycarbonyl-1 hydroxy-4 isobutyl-5) pyrrolidinone-2.

Composé (V) : W' = Boc, $R_1 = CH_2CH(CH_3)_2$.

Le produit brut obtenu à l'étape précédente est repris par 100 ml de $CH_2Cl_2$ auxquels on ajoute 8 ml d'acide acétique. La solution est agitée dans un bain de glace, puis on ajoute, par petites portions, pendant 1 h, 1,4 g de borohydrure de sodium et on laisse 4 h sous forte agitation. Le milieu réactionnel est hydro lysé par de la glace, on maintient le pH de la solution aqueuse entre 3 et 4 par addition d'acide chlorhydrique 0,5 N. La phase organique est lavée à l'eau, séchée sur sulfate de magnésium et évaporée. Le produit réduit est purifée par chromatographie sur 300 ml de gel de silice, en éluant par un mélange équivolumique hexane-acétate d'éthyle. On obtient 3,6 g du composé attendu.

Rendement : 70 % par rapport à la Boc Leucine hydratée de départ ;

Fc = 90-91°C ;

Rf : (Hexane/AcOEt : 1/3) = 0.58 ;

alpha D = +63.

SPECTRE DE RMN

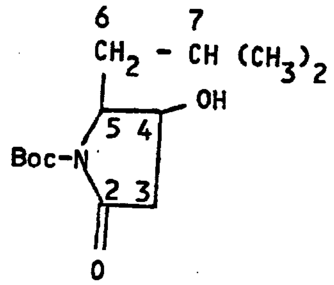

| Delta | Aspect | Intégration | Attribution |
|---|---|---|---|
| 1,45 | s | 9 H | $(CH_3)_3$ |
| 2,45 | qd | 2 H | $CH_2$ en 3 |
| 4,30 | q | 1 H | CH en 4 |
| 4,0 | m | 1 H | CH en 5 |
| 5,20 | | 1 H | OH |
| 1,38 | m | 1 H) | |
| | | ) | $CH_2$ en 6 |
| 2,75 | m | 1 H) | |
| 2,75 | m | 1 H | CH en 7 |
| 0,90 | dd | 6 H | $(CH_3)_2$ |

5) Acide (3S,4S) tert-butyloxycarbonylamino-4 hydroxy-3 méthyl-3 heptanoïque.

Le produit obtenu à l'étape précédente est repris par 15 ml d'acétone, on ajoute goutte à goutte 14 ml de soude N. L'hydrolyse, suivie en CCM, est terminée après 5 min de réaction. On porte à pH 3-4 par addition d'acide chlorhydrique 1 N, puis on ajoute 200 ml d'acétate d'éthyle et 100 ml d'eau. La solution organique est lavée à l'eau, séchée sur sulfate de magnésium et évaporée. Le produit brut de la réaction est repris par 15 ml d'acétone puis cristallisé par addition de 100 ml d'hexane. On obtient 3,48 g de poudre blanche.

Rendement : 63 % par rapport à la Boc Leucine hydratée de départ ;

Rf : ($CH_2Cl_2$/MeOH/AcOH : 93/5/2) = 0,43 ;

alpha D = -41°C.

SPECTRE DE RMN

| Delta | Aspect | Intégration | Attribution |
|---|---|---|---|
| 1,36 | s | 9 H | $(CH_3)_3$ |
| 2,22 | q | 2 H | $CH_2$ |
| 3,81 | m | 1 H | CH - OH |
| 3,50 | m | 1 H | CH - NH |
| 6,22 | d | 1 H | NH |
| 1,55 | m | 1 H | CH |
| 1,26 | m | 2 H | $CH_2$ |
| 0,85 | d | 6 H | $(CH_3)_2$ |

EXEMPLE 2 :

Ester méthylique de l'acide (3S,4S) tert-butyloxycarbonylamino-4 hydroxy-3 méthyl-6 heptanoïque.

Ce composé est obtenu à partir de celui préparé à l'exemple 1, étape 4 : (S,S) tert-butyloxycarbonyl-1 hydroxy-4 isobutyl-5 pyrrolidinone-2.

257 g de ce produit sont dissous dans 5 ml de méthanol puis on ajoute 0,6 ml d'une solution 2 N de méthanolate de sodium. Après 10 min à température ambiante, la solution est concentrée, reprise par une solution aqueuse de sulfate acide de potassium à 5 %, puis extraite par l'acétate d'éthyle. La phase organique est lavée à l'eau, séchée sur sulfate de sodium et évaporée. Après passage sur une colonne de silice éluée par de l'éther, le produit attendu est cristallisé dans l'hexane à froid. On obtient 260 mg.

Rendement : 90 % ;

Rf : (hexane/AcOt : 1/1) = 0,77 ;

alpha D = -40 ;

Fc = 57-58°C.

SPECTRE DE RMN

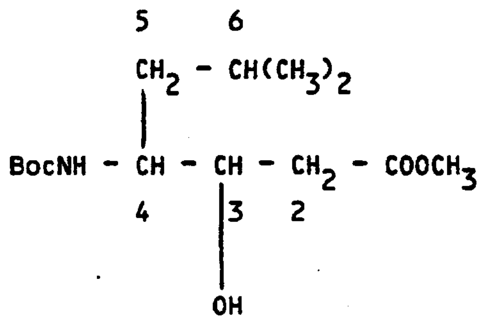

| Delta | Aspect | Intégration | Attribution |
|---|---|---|---|
| 1,37 | s | 9 H | $(CH_3)_3$ |
| 6,28 | d | 1 H | NH |
| 3,83 | m | 1 H | CHOH |
| 3,51 | m | 1 H | CHNH |
| 4,78 | d | 1 H | OH |
| 3,57 | s | 3 H | $OCH_3$ |
| 2,30 | qd | 2 H | $CH_2$ en 2 |
| 1,25 | m | 2 H | $CH_2$ en 5 |
| 1,54 | m | 1 H | CH en 6 |
| 0,84 | dd | 6 H | $(CH_3)_2$ |

EXEMPLE 3 :

Acide (3S,4S) tert-butyloxycarbonylamino-4 hydroxy-3 phényl-5 pentanoïque.

1) (S) ((tert butyloxycarbonylamino-2 hydroxy-1 phényl-3) propylidène)-5 diméthyl-2,2 dioxanne-1,3 dione-4,6.

Composé (III) : W' = Boc, $R_1 = CH_2C_6H_5$.

Dans 30 ml de dichlorométhane, on mélange 2,65 g de Boc-Phe-Oh, 2,7 g de diméthylaminopyridine et 1,6 g d'acide de Meldrum. La solution est portée à -7°C et on ajoute lentement, pendant 1 h, 1,3 ml de chloroformiate d'isopropényle dans 20 ml de dichlorométhane. Après 2 h à -7°C, on lave par une solution de sulfate acide de potassium à 5 % puis par une solution de chlorure de sodium saturée. L'évaporation du solvant conduit à la formation d'un précipité blanc ; la condensation est quantitative. Le produit obtenu est utilisé tel quel ou après purification. Le précipité est repris par une solution d'un mélange équivolumique de méthanol et éther. On obtient 3 g du produit attendu sous forme d'un précipté blanc. Il reste du produit non purifié dans les eaux mères.

Rendement : 77 %.

Le produit attendu peut également être préparé en présence d'un agent de couplage. Dans 20 ml de dichlorométhane, on mélange 1,33 g de Boc-Phe-OH, 750 mg d'acide de Meldrum et 900 mg de diméthylamino-4 pyridine à température ambiante et on ajoute 1,2 g de dicyclohexylcarbodiimide. On observe la formation rapide d'un précipité blanc et le milieu réactionnel devient jaune. Après 3 h à température ambiante, le milieu est concentré et repris par de l'acétate d'éthyle, le précipité est filtré, la solution est lavée par du sulfate acide de potassium à 5 % puis par une solution saline saturée, séchée sur sulfate de sodium

et évaporée. Le mélange et repris par une solution équivolumique de méthanol et éther. On obtient 300 mg du produit attendu sous forme de précipité blanc.

Rendement : 15 % ;

Fc = 120-121°C ;

alpha D : (DMF, c = 1) = +88 ;

Rf : (AcOEt/MeOH/AcOH : 95/3/2) = 0,71.

## SPECTRE DE RMN

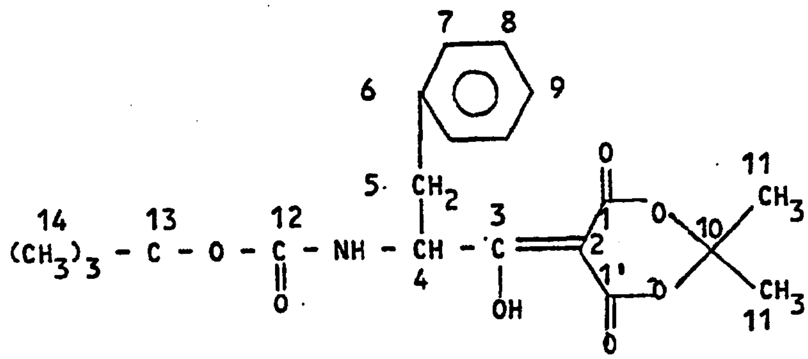

| Delta | Aspect | Intégration | Attribution |
|---|---|---|---|
| 11,66 | s large | 1 H | OH |
| 5,57 | m | 1 H | H en 4 |
| 2,88 | m | 1 H | H en 5 |
| 7,36 | d | 2 H | ) |
| 7,28 | t | 2 H | )$C_6H_5$ |
| 7,20 | t | 1 H | ) |
| 7,42 | d | 1 H | NH |
| 1,29 | s | 9 H | $C(CH_3)_3$ |
| 1,66 | s | 6 H | $(CH_3)_2$ |

SPECTRE DE RMN du Carbone 13

| Delta | Aspect | Attribution |
|---|---|---|
| 165 | s large | $C_1$ |
| 90 | s | $C_2$ |
| 195 | s | $C_3$ |
| 55 | d | $C_4$ |
| 47 | t | $C_5$ |
| 137 | s | $C_6$ |
| 128 | d | $C_8$ |
| 130 | d | $C_7$ |
| 126 | d | $C_9$ |
| 105 | s | $C_{10}$ |
| 26 | q | $C_{11}$ |
| 155 | s | $C_{12}$ |
| 78 | s | $C_{13}$ |
| 27 | q | $C_{14}$ |

2) (S) tert-butyloxycarbonyl-1 hydroxy-4 benzyl-5 1,2-dihydropyrrolone-2.

Composé (IV-IVa) : W' = Boc et $R_1 = CH_2C_6H_5$

800 mg du composé obtenu à l'étape précédente sont chauffés dans 20 ml de méthanol à reflux pendant 20 min. après évaporation du solvant, le produit et dissous dans l'éther et cristallisé par addition d'hexane. On obtient 570 mg du produit attendu.

Rendement : 95 % soit 88 % par rapport à la Boc-PHe-OH de départ ;
Fc = 141-142°C ;
alpha D = +230 ;
Rf : (AcOET/MeOH/AcOH : 95/3/2) = 0,48.

SPECTRE DE RMN

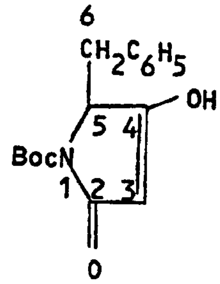

| Delta | Aspect | Intégration | Attribution |
|---|---|---|---|
| 1,5 | s | 9 H | $C(CH_3)_2$ |
| 4,66 | s | 1 H | CH en 3 |
| 4,60 | q | 1 H | CH en 5 |
| 3,21 | m | 2 H | $CH_2$ en 6 |
| 7,0 | m | 2 H | ) |
| | | | ) $C_6H_5$ |
| 7,2 | m | 3 H | ) |

3) (S,S) tert-butyloxycarbonyl-1 hydroxy-4 benzyl-5 pyrrolidinone-2.

Composé (V) : W' = Boc et $R_1$ = $CH_2C_6H_5$

Ce composé est obtenu en suivant la mode opératoire décrit à l'exemple 1, étape 4.

Fc 120-122°C ;

alpha D = +43 ;

Rf : (hexane/AcOEt : 1/3) = 0,58.

SPECTRE DE RMN

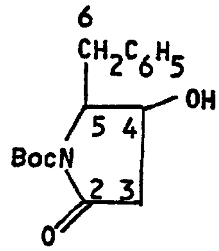

| Delta | Aspect | Intégration | Attribution |
|---|---|---|---|
| 1,35 | s | 9 H | $(CH_3)_3$ |
| 2,37 | qd | 2 H | $CH_2$ en 3 |
| 4,30 | m | 1 H | CH en 4 |
| 4,25 | m | 1 H | CH en 5 |
| 5,5 | d | 1 H | OH |
| 3,0 | qd | 2 H | $CH_2$ en 6 |
| 7,25 | m | 5 H | $C_6H_5$ |

4) Acide (3S,4S) tert-butyloxycarbonylamino-4 hydroxy-3 phényl-5 pentanoïque.

Composé (I) : W = Boc, $R_1 = CH_2C_6H_5$, $R_2$ = H.

Ce composé et obtenu en suivant le mode opératoire décrit à l'exemple 1, étape 5.

alpha D = -36 ;

Rf : ($CH_2Cl_2$/MeOH/AcOH : 95/5/2) = 0,44.

SPECTRE DE RMN

| Delta | Aspect | Intégration | Attribution |
|---|---|---|---|
| 1,29 | s | 9 H | $C(CH_3)_3$ |
| 2,29 | qd | 2 H | $CH_2$ |
| 3,88 | m | 1 H | CH - OH |
| 3,66 | m | 1 H | CH - NH |
| 6,49 | d | 1 H | NH |
| 2,7 | qd | 2 H | $CH_2-C_6H_5$ |
| 7,2 | m | 5 H | $C_6H_5$ |

EXEMPLE 4 :

Acide (3R,4R) tert-butyloxycarbonylamino-4 hydroxy-3 phényl-5 pentanoïque.

Ce produit est préparé en suivant le mode opératoire décrit à l'exemple 3 à partir de la (D) Boc-Phe-OH. Les produits obtenus à chaque étape se distinguent de ceux obtenus aux étapes correspondantes de l'exemple

précédent par des pouvoirs rotatoires opposés.

1) (R) tert-butyloxycarbonylamino-2 hydroxy-1 phényl-3 propylidène)-5 diméthyl-2,2 dioxanne-1,3 dione-4,6.

Composé (III) : W' = Boc, $R_1$ = $CH_2C_6H_5$ ;
Fc = 118-120°C ;
alpha D : (DMF, c = 1) = -87,5.

2) (R) tert-butyloxycarbonyl-1 hydroxy-4 benzyl-5 1,2-dihydropyrrolone-2.

Composé (IV-IVa) : W' = Boc, $R_1$ = $CH_2C_6H_5$ ;
Rf : (AcOEt/MeOH/AcOH : 95/3/2) = 0,48 ;
Fc = 148-149°C ;
alpha D = -230.

3) (R,R) tert-butyloxycarbonyl-1 hydroxy-4 benzyl-5 pyrrolidinone-2.

Composé (V) : W' = Boc, $R_1$ = $CH_2C_6H_5$.
Rf : (hexane/AcOEt) = 0,58 ;
Fc = 119-120°C ;
alpha D = -43,5.

4) Acide (3R,4R) tert-butyloxycarbonylamino-4 hydroxy-3 phényl-5 pentanoïque.
Rf : ($CH_2Cl_2$/MeOH/AcOH : 93/5/2) = 0,44 ;
Fc = 146-148°C ;
alpha D = +35.

EXEMPLE 5 :

Ester méthylique de l'acide (3S,4S) benzyloxycarbonylamino-4 hydroxy-3 pentanoïque.

1) (S) benzyloxycarbonyl-1 hydroxy-4 méthyl-5 1,2-dihydropyrrolone-2.

Composé (IV-IVa) : W' = Z, $R_1$ = $CH_3$.
Ce composé est préparé en suivant la méthode décrite à l'exemple 1, étape 3.
Rendement calculé par rapport à l'acide amino de départ (Z Ala OH) : 89 %.
Rf : (AcOEt/MeOH/AcOH : 95/3/2) = 0,33 ;
alpha D = +60,5.

SPECTRE DE RMN

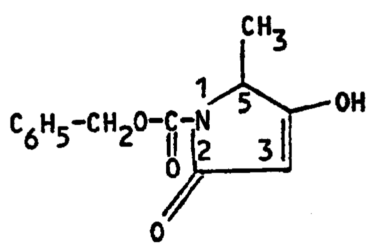

| Delta | Aspect | Intégration | Attribution |
|---|---|---|---|
| 7,4 | m | 5 H | $C_6H_5$ |
| 5,22 | q | 2 H | $CH_2$ |
| 4,88 | s | 1 H | CH en 3 |
| 4,41 | q | 1 H | CH en 5 |
| 12,38 | s | 1 H | OH |
| 1,39 | d | 3 H | $CH_3$ |

2) (S) benzyloxycarbonyl-1 hydroxy-4 méthyl-5 pyrrolidinone-2.

Composé (V) : W = Z, $R_1 = CH_3$.

Ce composé est préparé en suivant la méthode décrite à l'exemple 1, étape 4.

Rendement calculé par rapport à l'acide aminé de départ : 58 %.

Rf : (hexane/AcOEt : 1/3) = 0,36 ;

alpha D = +50.

**SPECTRE DE RMN**

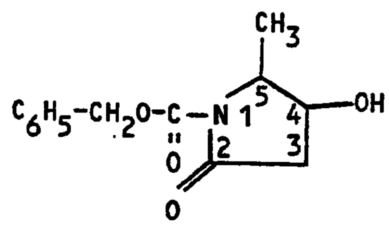

| Delta | Aspect | Intégration | Attribution |
|---|---|---|---|
| 7,40 | m | 5 H | $C_6H_5$ |
| 5,21 | q | 2 H | $CH_2$ |
| 2,54 | qd | 2 H | $CH_2$ en 3 |
| 4,82 | q) | 1 H | CH en 4 |
| | )J=6,6 Hz | | |
| 4,15 | m) | 1 H | CH en 5 |
| 5,35 | | 1 H | OH |
| 1,2 | d | 3 H | $CH_3$ |

3) Ester méthylique de l'acide (3S,4S) benzyloxycarbonylamino-4 hydroxy-3 pentanoïque.

Composé (I) : W = Z, $R_1 = CH_3$, $R_2 = CH_3$.

500 g du produit obtenu à l'étape précédente sont repris dans 5 ml de méthanol, on ajoute 1,5 ml d'une solution de méthanol chlorhydrique environ 2N. Le milieu réactionnel et porté à reflux pendant 20 min puis le solvant est évaporé et le produit de la réaction est repris dans du méthanol et évaporé pour chasser l'excès d'acide. Après chromatographie sur une colonne de gel de silice éluée par de l'éther, le produit est cristallisé dans un mélange éther-hexane à 8/2 en volume. On obtient 525 mg du composé attendu.

Rendement 93 % ;

Rf : hexane/AcOEt : 1/1) = 0,44 ;

Fc = 90-92°C ;

alpha D = -17,5.

SPECTRE DE RMN

| Delta | Aspect | Intégration | Attribution |
|---|---|---|---|
| 7,35 | m | 5 H | $C_6H_5$ |
| 5,02 | s | 2 H | $CH_2-C_6H_5$ |
| 6,96 | d | 1 H | NH |
| 3,90 | m | 1 H | CHOH |
| 3,62 | m | 1 H | CHNH |
| 4,81 | large | 1 H | OH |
| 3,58 | s | 3 H | $OCH_3$ |
| 2,35 | qd | 2 H | $CH_2-CO_2$ |
| 1,03 | d | 2 H | $CH_3$ |

EXEMPLE 6

Acide (3S,4S) benzyloxycarbonylamino-4 hydroxy-3 pentanoïque.

Ce composé est préparé à partir du produit obtenu à l'exemple 5, étape 2. 1,25 g de ce composé sont repris par 20 ml de dioxanne, on ajoute 5 ml d'acide chlorhydrique 1N puis on chauffe à reflux pendant 2 h. Le solvant est concentré, le milieu réactionnel est repris par de l'eau et extrait par l'acétate d'éthyle ; la solution organique est extraite par 50 ml de bicarbonate de sodium à 5 %. La phase aqueuse est neutralisée par de l'acide citrique en poudre et extraite par de l'acétate d'éthyle. La phase organique est lavée à l'eau puis par une solution saturée de chlorure de sodium, séchée et évaporée. On obtient 1,1 g du composé attendu.

Rendement 83 % ;

Rf ($CH_2Cl_2$/MeOH/AcOH : 93/5/2) = 0,27 ;

alpha D = -15.

SPECTRE DE RMN

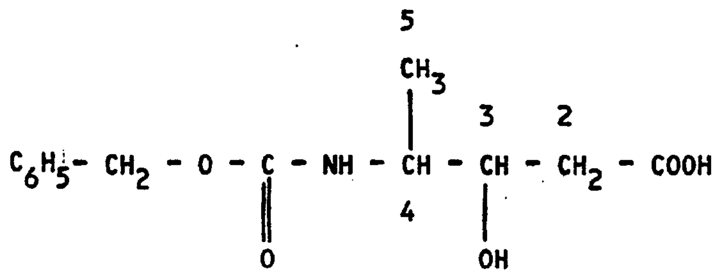

| Delta | Aspect | Intégration | Attribution |
|---|---|---|---|
| 7,40 | m | 5 H | $C_6H_5$ |
| 5,22 | q | 2 H | $CH_2$ |
| 2,53 | qd | 2 H | $CH_2$ en 2 |
| 4,32 | m | 1 H | CH en 3 |
| 4,16 | m | 1 H | CH en 4 |
| 5,36 | d | 1 H | NH |
| 1,22 | d | 3 H | $CH_3$ |

En utilisant les mêmes méthodes que dans les exemples précédents, on a préparé les composés (I) selon l'invention décrits dans le tableau 1. Ces composés, ayant été obtenus à partir d'acides aminés naturels de configuration L, ont tous la configuration (3S,4S). Ces produits ont été caractérisés par leur pouvoir rotatoire (alpha D), leur Rf, éventuellement leur point de fusion (Fc), (tableau 2) et par leur spectre de RMN (tableau 3, pour les composés dans lesquels $R_2$ = H et tableau 4, pour les composés dans lesquels $R_2$ = $CH_3$).

## TABLEAU 1

Composé (I) : (3S,4S) WNH-C*H-C*H-$CH_2$-$COOR_2$
$\quad\quad\quad\quad\quad\quad\quad\quad\quad\quad\quad\quad$ | $\quad$ |
$\quad\quad\quad\quad\quad\quad\quad\quad\quad\quad\quad\quad$ $R_1$ $\quad$ OH

| N° Exemple | W | $R_1$ | $R_2$ |
|---|---|---|---|
| 7 | Boc | $CH(CH_3)_2$ | H |
| 8 | " | " | $CH_3$ |
| 9 | " | $CH_2C_6H_5$ | $CH_3$ |
| 10 | " | $CH_2$-O-$CH_2C_6H_5$ | H |
| 11 | " | " | $CH_3$ |
| 12 | " | $CH_2CH_2$-S-$CH_3$ | H |
| 13 | " | " | $CH_3$ |
| | " | | |
| 14 | " | -$CH_2$- 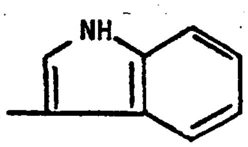 | H |
| 15 | " | " | $CH_3$ |
| 16 | " | $(CH_2)_4$ NHZ | H |
| 17 | " | " | $CH_3$ |
| 18 | " | $CH_2C_6H_{11}$ | H |

## TABLEAU 2

Caractéristiques physico-chimiques des composés (I)

| N° Exemple | Rf | Fc °C | alpha D |
|---|---|---|---|
| 7 | a) 0,45 | | - 43 |
| 8 | b) 0,74 | | c) - 42 |
| 9 | b) 0,66 | 97 - 98 | - 35 |
| 10 | a) 0,47 | | - 3,5 |
| 11 | b) 0,69 | | - 5 |
| 12 | a) 0,40 | 107 - 108 | - 30,6 |
| 13 | b) 0,56 | | - 31 |
| 14 | a) 0,32 | 162 - 164 | - 39 |
| 15 | b) 0,56 | | - 31 |
| 16 | a) 0,40 | 99 - 100 | - 19,5 |
| 17 | b) 0,35 | | - 19 |
| 18 | d) 0,17 | 100 - 101 | - 35,20 |

a) Rf ($CH_2Cl_2$/MeOH/AcOH : 93/5/2)
b) Rf (Hexane/AcOEt : 1/1)
c) (0,5 molaire dans MeOH)
d) Rf (AcOEt/MeOH : 9/1)

## TABLEAU 3

**Spectre RMN des composés (I) dans lesquels $R_2$ = H**

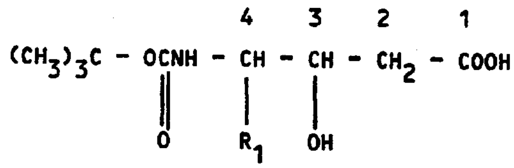

| N° Exemple | Analyse des déplacements chimiques Delta: $(CH_3)_3$ 9H s | $H_2$ 2H qd | $H_3$ 1H m | $H_4$ 1H m | NH 1H d | OH 1H d | $R_1$ |
|---|---|---|---|---|---|---|---|
| 7 | 1,37 | 2,22 | 4,02 | 3,04 | 6,06 | | 1H m 1,71<br>3H d 0,89<br>3H d 0,82 |
| 10 | 1,38 | 2,30 | 4,05 | 3,70 | 6,34 | | 5H m 7,32<br>2H t 4,46<br>2H q 3,44 |
| 12 | 1,37 | 2,24 | 3,86 | 3,51 | 6,39 | | 2H m 2,43<br>3H s 2,02<br>2H m 1,71<br>et 1,56 |
| 14 | 1,37 | 2,31 | 3,97 | 3,75 | 6,44 | | 1H s 10,75<br>1H d 7,56<br>1H d 7,3<br>1H s 7,10<br>1H t 7,06<br>1H t 6,98<br>2H qd 2,84 |
| 16 | 1,37 | 2,24 | 3,86 | 3,38 | 6,26 | | 5H m 7,33<br>1H m 7,2<br>2H s 5,0<br>2H m 2,98<br>4H m 1,44<br>2H m 1,26 |
| 18 | 1,34 | 2 à 2,48 (a) | | 2,93 à 3,78 (b) | 6,21 | | m 0,62 à 1,78 |

(a) m complexe avec CH en 3

(b) m complexe avec CH du cyclohexyle

TABLEAU 4

Spectre RMN des composés (I) dans lesquels $R_2 = CH_3$

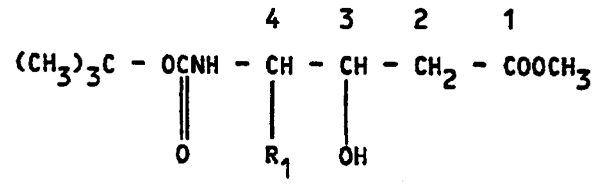

| N° Ex | Analyse des déplacements chimiques Delta (CH$_3$)$_3$ 9H s | H$_2$ 2H q | H$_3$ 1H m | H$_4$ 1H m | NH 1H d | OH 1H d | OCH$_3$ 3H s | R$_1$ |
|---|---|---|---|---|---|---|---|---|
| 8 | 1,38 | 2,32 | 4,05 | 3,04 | 6,10 | 4,64 | 3,57 | 1H m 1,72 |
| | | | | | | | | 3H d 0,89 |
| | | | | | | | | 3H d 0,82 |
| 11 | 1,38 | 2,37 | 4,06 | 3,69 | 6,38 | 4,88 | 3,58 | 5H m 7,32 |
| | | | | | | | | 2H s 4,46 |
| | | | | | | | | 2H q 3,43 |
| 13 | 1,37 | 2,30 | 3,89 | 3,51 | 6,45 | 4,88 | 3,57 | 2H m 2,41 |
| | | | | | | | | 3H s 2,01 |
| | | | | | | | | 2H m 1,71 |
| | | | | | | | | et 1,56 |
| 15 | 1,36 | 2,40 | 3,99 | 3,77 | 6,45 | 4,99 | 3,56 | 1H s 7,62 |
| | | | | | | | | 1H d 7,59 |
| | | | | | | | | 1H d 7,34 |
| | | | | | | | | 1H s 7,12 |
| | | | | | | | | 1H t 7,07 |
| | | | | | | | | 1H t 6,98 |
| | | | | | | | | 2H q 2,85 |
| 17 | 1,36 | 2,30 | 3,88 | 3,37 | 6,31 | 4,83 | 3,58 | 5H m 7,32 |
| | | | | | | | | 1H m 7,21 |
| | | | | | | | | 2H s 5,0 |
| | | | | | | | | 2H m 2,98 |
| | | | | | | | | 4H m 1,45 |
| | | | | | | | | 2H m 1,25 |

L'obtention des composés (I) décrits dans les exemples 7 à 18 a été réalisée par la préparation intermédiaire des composés (IV-IVa) et (V) correspondants. Les résultats relatifs aux composés (IV-IVa) sont rassemblés dans le tableau 5 qui donne pour chacun d'eux le rendement par rapport à l'acide aminé de départ (II), le pouvoir rotatoire (alpha D), éventuellement le point de fusion (Fc) et le Rf du composé (IV, (IVa) et du composé (III) obtenu à l'étape immédiatement précédente.

Le spectre de RMN de ces composés est décrit dans le tableau 6. Les résultats relatifs aux composés (V) sont rassemblés dans le tableau 7 qui donne pour chacun d'eux le rendement par rapport à l'acide aminé de départ (II), le Rf, le pouvoir rotatoire (alpha D) et éventuellement le point du fusion (Fc). Le spectre de RMN de ces composés est décrit dans le tableau 8.

## TABLEAU 5

### Caractéristiques physico-chimiques des composés (IV-IVa)

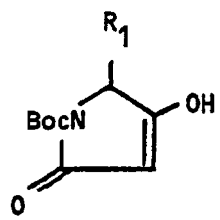

| N° Ex | $R_1$ | Rendement (II ---- IV) | Rf AcOEt/MeOH/AcOH 95/3/2 Composé (III) | Rf AcOEt/MeOH/AcOH 95/3/2 Composé (IV-IVa) | alpha D | Fc °C |
|---|---|---|---|---|---|---|
| 7 | $CH(CH_3)_2$ | 91 % | 0,52 | 0,42 | + 123 | 120-121 |
| 10 a) | $CH_2OCH_2C_6H_5$ | 80 % | 0,68 | 0,46 | + 55 | - |
| 12 | $CH_2CH_2SCH_3$ | 85 % | 0,61 | 0,44 | | - |
| 14 | $CH_2$- 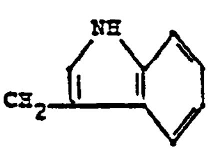 | 86 % | 0,62 | 0,42 | + 229 | 96-98 |
| 16 | $(CH_2)_4$ NHZ | 82 % | 0,61 | 0,28 | + 72 | - |

a) Ce produit est sous forme de sel de sodium

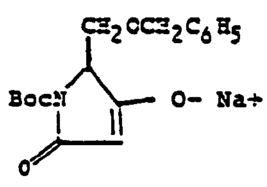

TABLEAU 6

Spectre RMN des composés (IV-IVa)

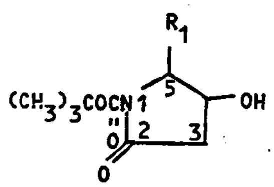

| N° Ex | Analyse des déplacements chimiques Delta: $(CH_3)_3$ 9H s | $H_3$ 1H s | $H_5$ 1H m | OH 1H s | $R_1$ |
|---|---|---|---|---|---|
| 7 | 1,42 | 4,85 | 4,25 | 6,19 | 1H m 2,33 |
| | | | | | 3H d 1,02 |
| | | | | | 3H d 0,76 |
| 10 | 1,40 | 4,91 | 4,40 | 12,29 | 2H m 3,85 |
| | | | | | 2H q 4,47 |
| | | | | | 5H m 7,27 |
| a) 10 | 1,36 | 3,89 | 3,65 | - | 2H m 3,78 |
| | | | | | 2H q 4,43 |
| | | | | | 5H m 7,27 |
| 12 | 1,45 | 4,88 | 4,46 | 12,38 | 2H m 2,0 |
| | | | | | 2H m 2,28 |
| | | | | | 3H s 1,98 |
| 14 | 1,5 | 4,60 | 4,63 | 12,25 | 2H m 3,40 |
| | | | | | 1H s 6,89 |
| | | | | | 1H t 6,95 |
| | | | | | 1H t 7,05 |
| | | | | | 1H d 7,32 |
| | | | | | 1H d 7,50 |
| | | | | | 1H s 10,85 |
| 16 | 1,43 | 4,87 | 4,38 | 12,26 | 2H m 1,77 |
| | | | | | 2H m 1,37 |
| | | | | | 1H t 7,20 |
| | | | | | 2H m 2,95 |
| | | | | | 2H s 4,98 |
| | | | | | 5H m 7,32 |

a) Pour ce composé, on a également enregistré le spectre en milieu basique, le composé étant alors sous forme de sel de sodium

TABLEAU 7

Caractéristiques physico-chimiques des compsés (V)

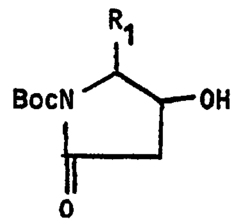

| N° Ex | $R_1$ | Rendement (II)--(V) | Rf Hexane/AcOEt 1/3 | alpha D | Fc °C |
|---|---|---|---|---|---|
| 7 | $CH(CH_3)_2$ | 57 % | 0,55 | + 60 | 99-1( |
| 10 | $CH_2OCH_2C_6H_5$ | 78 % | 0,52 | + 59 | 100-1( |
| 12 | $CH_2CH_2SCH_3$ | 68 % | 0,51 | | |
| 14 | $CH_2$ 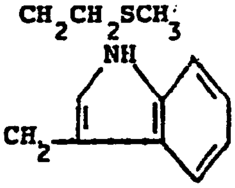 | 70 % | 0,38 | + 7 | 159-1( |
| 16 | $(CH_2)_4NHZ$ | 60 % | 0,31 | + 26 | |
| 18 | $CH_2C_6H_{11}$ | 35,2 % | a) 0,58 | + 53,2 | 94 |

a) Rf (acétate d'éthyle)

## TABLEAU 8

### Spectre RMN des composés (V)

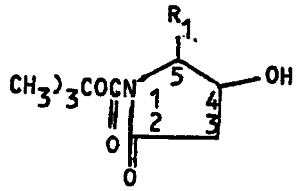

| N° Ex | Analyse des déplacements chimiques Delta | | | | | | |
|---|---|---|---|---|---|---|---|
| | $(CH_3)_3$ 9H s | $H_3$ 2H qd | $H_4$ 1H m | $H_5$ 1H m | OH 1H d | $R_1$ | |
| 7 | 1,43 | 2,43 | 4,46 | 3,90 | 5,35 | 6H dd | 0,95 |
| 10 | 1,40 | 2,50 | 4,45 | 4,10 | 5,45 | 2H q | 3,77 |
| | | | J = 7 Hz | | | 2H q | 4,48 |
| | | | | | | 5H m | 7,30 |
| 12 | 1,44 | 2,48 | 4,33 | 4,04 | 5,38 | 2H m | 2,02 |
| | | | | | | | à 1,88 |
| | | | | | | 2H m | 2,51 |
| | | | | | | 3H s | 2,05 |
| 14 | 1,35 | 2,40 | 4,30 | 4,30 | 5,49 | 2H q | 3,15 |
| | | | | | | 1H d | 7,10 |
| | | | | | | 1H d | 7,6 |
| | | | | | | 1H t | 7,5 |
| | | | | | | 1H t | 6,98 |
| | | | | | | 1H d | 7,92 |
| | | | | | | 1H | 7,57 |
| 16 | 1,43 | 2,46 | 4,31 | 3,92 | 5,30 | 2H m | 1,66 |
| | | | | | | 4H m | 1,33 |
| | | | | | | 2H q | 3,0 |
| | | | | | | 5H m | 7,34 |
| | | | | | | 1H t | 7,2 |
| | | | | | | 2H s | 5,0 |
| 18 | 1,38 | 2,40 | 4,25 | 4,0 | 5,25 | 13H m | 0,71 |
| | | | | | | | à 1,82 |

EXEMPLE 19 :

Trifluoroacétate de l'ester méthylique de l'acide (3S,4S)- amino-4 hydroxy-3 méthyl-6 hexanoïque.

A partir de 486 mg du composé (V) (W' = Boc, $R_1$ : $CH(CH_3)_2$) tel que décrit dans les tableaux 7 et 8 (exemple 7), on prépare le composé (I) de l'exemple 8 décrit dans les tableaux 2 et 4.

On prépare ensuite le composé (I) dans lequel W = H, de la façon suivante : la réaction est effectuée par addition de 1 ml de solution de méthanolate de sodium 2N dans le méthanol puis, après 10 min à température ambiante, addition de méthanol chlorhydrique, le solvant est évaporé ; le produit de la réaction est repris par 10 ml de dichlorméthane puis la solution est filtrée et on ajoute 2,5 ml d'acide trifluoroacétique. Après 30 min à température ambiante, le milieu réactionnel est concentré et le sel de trifluoroacétate est précipité par addition d'éther. Le précipité est ensuite filtré, rincé par de l'éther et séché au dessiccateur. On obtient 440 mg du composé attendu.

Rendement : 80 % ;
Fc = 140-142°C ;
alpha D = -1.

SPECTRE DE RMN

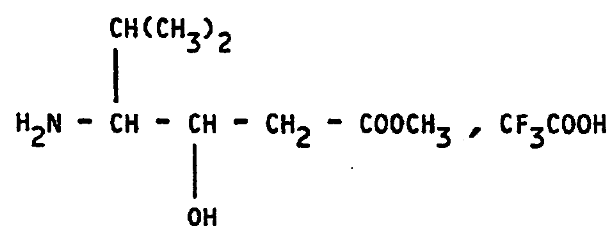

| Delta | Aspect | Intégration | Attribution |
|---|---|---|---|
| 7,72 | s | 2 H | $NH_2$ |
| 4,11 | m | 1 H | CH OH |
| 2,81 | q | 1 H | CH $NH_2$ |
| 5,81 | d | 1 H | OH |
| 3,61 | s | 3 H | $OCH_3$ |
| 2,61 | q | 2 H | $CH_2$ |
| 1,91 | m | 1 H | $CH(CH_3)_2$ |
| 0,96 | d | 3 H | $CH_3$ |
| 0,92 | d | 3 H | $CH_3$ |

**Revendications**

1. Procédé de préparation stéréospécifique de dérivés d'acides (3R, 4R)- ou, respectivement (3S, 4S)-amino-4 hydroxy-3 carboxyliques optiquement purs de formule:

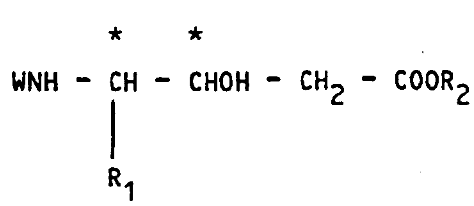

(I)

dans laquelle:

- W et l'hydrogène ou un groupe N-protecteur;
- $R_1$ représente un radical alkyle, linéaire ou ramifié ayant de 1 à 6 atomes de carbone, un radical méthyloxyalkyle inférieur, un radical benzyloxyalkyle inférieur, un radical méthylthioalkyle inférieur, un radical phénylthioalkyle inférieur, un radical benzylthioalkyle inférieur, un radical aminoalkyle inférieur libre ou substitué par un groupe protecteur sur la fonction amine, un radical (alkyle inférieur) aminoalkyle inférieur, un radical (dialkyle inférieur) aminoalkyle inférieur, un radical hydroxyalkyle inférieur, un radical carboxyalkyle inférieur libre ou estérifié, ou un radical carboxamido alkyle inférieur libre ou alkyle, un radical alkyle inférieur substitué à la fois et sur le même atome de carbone par une amine et un carboxy libre ou estérifié, un radical alcényle, linéaire ou ramifié, ayant de 2 à 6 atomes de carbone, un radical méthyloxyalcényle inférieur tel que méthyloxyvinyle, un radical phénoxyalcényle inférieur, un radical benzyloxyalcényle, un radical méthylthioalcényle inférieur, un radical phénylthioalcényle inférieur, un radical benzylthioalcényle inférieur, un radical aminoalcényle inférieur libre ou protégé sur la fonction amine, un radical (alkyle inférieur) aminoalcényle inférieur, un radical (dialkyle inférieur) aminoalcényle inférieur, un radical carboxyalcényle libre ou estérifié, un radical carboxamido alcényle inférieur libre ou alkylé, un radical alcynyle linéaire ou ramifié ayant de 2 à 6 atomes de carbone, ou bien l'un des radicaux de formules:

  Cy-A-, Cy-O-A', ou $R_3$S-A'-

  dans laquelle:
- Cy représente un radical hydrocarboné aromatique ou alicyclique ou un radical hétérocyclique cntenant un atome d'oxygène ou de soufre ou un ou deux atomes d'azote, Cy étant éventuellement mono-, di- ou tri-substitué par des radicaux hydroxy, alkyle inférieur, alcoxy inférieur, trifluorométhyle, nitro ou halogéno,
- A représente une liaison simple ou un radical alkylène, linéaire ou ramifié, ayant de 1 à 5 atomes de carbone, ou un radical alcénylène linéaire ou ramifié ayant des 2 à 5 atomes de carbone.
- A' représente un radical alkylène, linéaire ou ramifié, ayant de 1 à 5 atomes de carbone ou un radical alcénylène, linéaire ou ramifié ayant de 2 à 5 atomes de carbone.
- $R_3$ représente un groupement S-protecteur;
- $R_2$ représente l'hydrogène, un métal alcalin ou alcalino-terreux, un alkyle inférieur, un benzyle non substitué ou substitué par un groupement alkyle inférieur, par un halogène ou par un groupement nitro;

caractérisé en ce que

(a) l'on fait réagir l'acide de Meldrum sur un aminoacide optiquement pur, de configuration D ou, respectivement, L, protégé de formule:

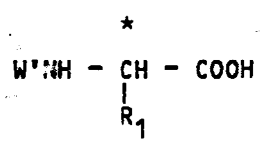

(II)

dans laquelle W' représente un groupe N-protecteur tel que Boc ou Z et $R_1$ a la signification indiquée plus haut, en milieu basique, en présence d'un agent activateur de l'acide amine (II) ou d'un agent de couplage;

(b) on chauffe ensuite le composé ainsi obtenu de formule (III);

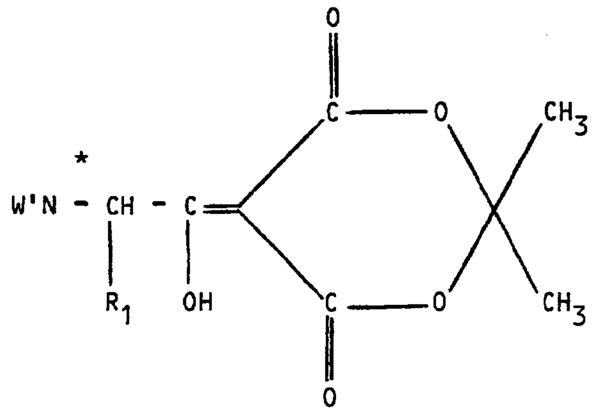

(III)

dans laquelle W' et $R_1$ sont tels que définis ci-dessus, dans un solvant à une température entre 30°C et 100°C;

(c) on réduit ensuite le composé (IV-IVa) ainsi obtenu sous 2 formes tautomères en équilibre dans lesquelles la chiralité de l'atome de carbone portant le substituant $R_1$ est conservée, représenté par les structures suivantes

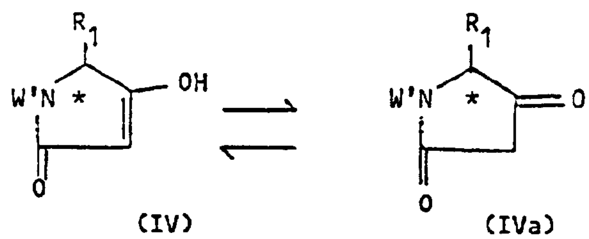

(IV) (IVa)

où W' et $R_1$ sont tels que définis ci-dessus, par un borohydrure métallique en milieu acide ou par une réduction catalytique sous pression d'hydrogène lorsque le substituant $R_1$ n'est pas sensible à l'hydrogénation.

(d) on ouvre le cycle du composé (V) ainsi obtenu de formule

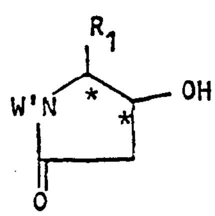

(V)

où W' et $R_1$ sont tels que définis ci-dessus soit en milieu acide lorsque W' représente un groupe N-protecteur stable en milieu acide, soit en milieu basique lorsque W' représente un groupe N-protecteur stériquement encombré et labile en milieu acide; et, si nécessaire, on effectue enfin une déprotection de l'azote par élimination du groupe N-protecteur du composé ainsi obtenu de formule

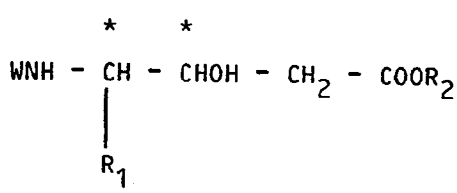

selon les méthodes connues.

2. Procédé selon la revendication 1, caractérisé en ce que

1) le composé de formule (II) est la Boc-L-leucine de formule :

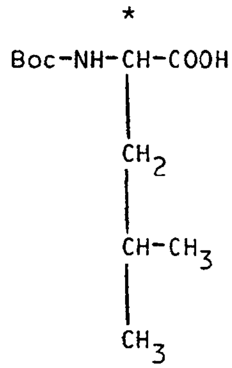

2) et en ce que l'étape d) et réalisée en milieu basique; en ce qu'éventuellement on effectue une déprotection de l'azote du composé ainsi obtenu et en ce que l'on isole l'acide (3S, 4S) amino-4 hydroxy-3 méthyl-6 heptanoïque, c'est-à-dire la statine.

**3.** Procédé selon la revendication 1, dans lequel $R_1$ représente un radical alkcle linéaire ou ramifié de 1 à 6 atomes de carbone, un benzyle, un cyclohexylméthyle, un méthylthioalkyle inférieur, un benzyloxyalkyle inférieur, un aminoalkyle inférieur libre ou substitué sur la fonction amine par un groupe N-protecteur, un indolyl-3 méthyle.

**4.** Dérivés d'acide (3R,4R) ou, respectivement, (3S,4S) amino-4-hydroxy-3-carboxyliques optiquement purs de formule :

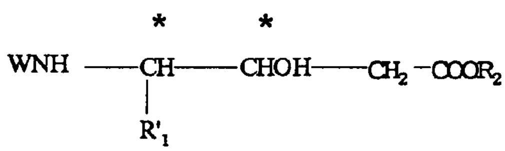

(VII)

dans laquelle W et $R_2$ ont les mêmes significations que dans la revendication 1, $R'_1$ a la même signification que $R_1$ à la condition que $R'_1$ soit autre que :

- alkyle en $C_1$-$C_6$ ;
- Cy-A- dans lequel Cy est un radical hydrocarboné aromatique ou alicyclique, et A est un alkylène en $C_1$-$C_5$ ;
- benzyle substitué par un nitro, oméga aminobutyle non substitué ou substitué par un groupe protecteur sur l'amine ;
- oméga amino-oméga carboxybutyle.

**5.** Composés optiquement purs selon la revendication 4, caractérisés en ce que $R'_1$ représente un radical méthylthioalkyle inférieur, un benzyloxyalkyle inférieur ou un indolyl-3-méthyle.

**6.** En tant qu'intermédiaires de synthèse utiles dans le procédé, selon l'une quelconque des revendications 1 à 3, les composés optiquement purs de fomule:

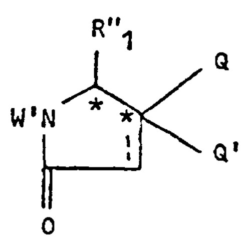

(VI)

dans laquelle W' est tel que défini dans la revendication 1, $R''_1$ a la même signification que $R_1$ de la re-

vendication 1 avec la limitation que R"$_1$ est autre que amino-4 butyle ou amino-4 carboxy-4 butyle, Q représente un groupe hydroxyle, Q' n'existe pas ou représente un atome d'hydrogène, ou bien Q et Q', pris ensemble, représentent un atome d'oxygène et la ligne en pointillé représente, lorsque Q' n'existe pas, une liaison supplémentaire, étant entendu que, lorsque Q' représente un atome d'hydrogène, le composé (VI) a la configuration (S, S) ou (R, R).

7. En tant qu'intermédiaire de synthèse utile dans le procédé, le composé optiquement pur selon la revendication 6, dans lequel R"$_1$ représente un radical alkyle linéaire ou ramifié de 1 à 6 atomes de carbone, un benzyle, un cyclohexylméthyle, un méthylthioalkyle inférieur, un benzyloxyalkyle inférieur, un indolyl-3 méthyle.

8. En tant qu'intermédiaires de synthèse utiles dans le procédé selon l'une quelconque des revendications 1 à 3, les composés optiquement purs de formule:

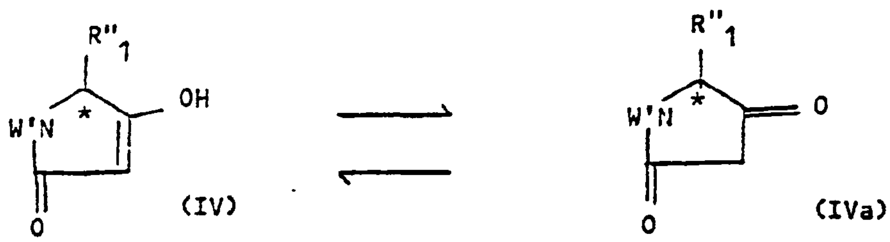

(IV) (IVa)

où W' a la même signification que dans la revendication 1 et R"$_1$ a la même signification que R$_1$ de la revendication 1 avec la limitation que R"$_1$ est différent de amino-4 butyle ou amino-4 carboxy-4 butyle.

9. En tant qu'intermédiaires de synthèse utiles dans le procédé selon l'une quelconque des revendications 1 à 3, les composés ayant la configuration (R, R) ou (S, S) de formule:

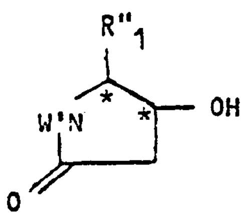

(V)

dans laquelle W' a la signification indiquée dans la revendication 1 et R"$_1$ a la même signification que R$_1$ de la revendication 1 avec la limitation que R"$_1$ est différent de amino-4 butyle ou amino-4 carboxy-4 butyle.

10. Composé optiquement pur de formule:

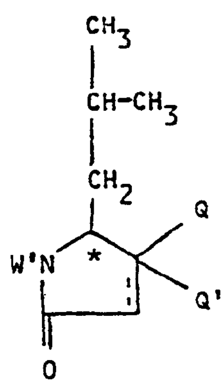

dans laquelle W' est tel que défini dans la revendication 1 et Q et Q' sont tels que définis dans la revendication 6.

**11.** Composé optiquement pur de formule:

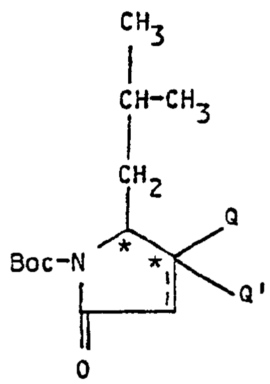

dans laquelle Q et Q' sont tels que définis dans la revendication 6.

**12.** Composé ayant la configuration (R, R) ou (S, S) de formule:

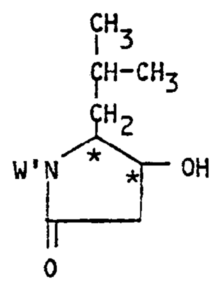

dans laquelle W' est tel que défini dans la revendication 1.

**13.** Composé ayant la configuration (R, R) ou (S, S) de formule:

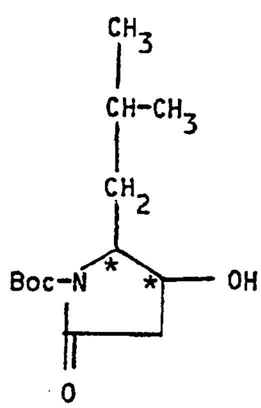

**14.** Composé selon l'une des revendications 4 à 10, ayant la configuration (S, S).

## Claims

**1.** Process for the stereospecific preparation of optically pure (3R,4R)-4-amino-3-hydroxycarboxylic or (3S,4S)-4-amino-3-hydroxycarboxylic acid derivatives of the formula:

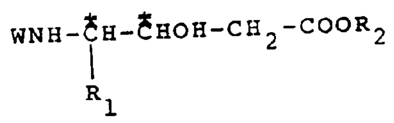 (I)

in which:

- W is hydrogen or an N-protecting group;
- $R_1$ represents a linear or branched alkyl radical having from 1 to 6 carbon atoms, a methoxy-lower alkyl radical, a benzyloxy-lower alkyl radical, a methylthio-lower alkyl radical, a phenylthio-lower alkyl radical, a benzylthio-lower alkyl radical, an amino-lower alkyl radical which is free or substituted by a protecting group on the amine group, a (lower alkyl)amino-lower alkyl radical, a (lower dialkyl)amino-lower alkyl radical, a hydroxy-lower alkyl radical, a free or esterified carboxy-lower alkyl radical, a free or alkylated carboxamido-lower alkyl radical, a lower alkyl radical substituted at the same time and on the same carbon atom by an amine and a free or esterified carboxyl, a linear or branched alkenyl radical having from 2 to 6 carbon atoms, a methoxy-lower alkenyl radical such as methoxyvinyl, a phenoxy-lower alkenyl radical, a benzyloxyalkenyl radical, a methylthio-lower alkenyl radical, a phenylthio-lower alkenyl radical, a benzylthio-lower alkenyl radical, an amino-lower alkenyl radical which is free or protected on the amine group, a (lower alkyl)amino-lower alkenyl radical, a (lower dialkyl)amino-lower alkenyl radical, a free or esterified carboxyalkenyl radical, a free or alkylated carboxamido-lower alkenyl radical or a linear or branched alkynyl radical having from 2 to 6 carbon atoms, or alternatively one of the radicals of the formulae:

  Cy-A- , Cy-O-A'- or $R_3$S-A'-

  in which:
  - ° Cy represents an aromatic or alicyclic hydrocarbon radical or a heterocyclic radical containing an oxygen or sulfur atom or one or two nitrogen atoms, Cy optionally being mono-, di-or tri-substituted by hydroxyl, lower alkyl, lower alkoxy, trifluoromethyl, nitro or halogeno radicals,
  - ° A represents a single bond or a linear or branched alkylene radical having from 1 to 5 carbon atoms,
  - ° A' represents a linear or branched alkylene radical having from 1 to 5 carbon atoms, and
  - ° $R_3$ represents an S-protecting group; and
- $R_2$ represents hydrogen, an alkali metal or alkaline earth metal, a lower alkyl or a benzyl which is unsubstituted or substituted by a lower alkyl group, a halogen or a nitro group,

characterized in that it consists in:

(a) reacting Meldrum's acid with a protected, optically pure amino acid, in the D or, respectively, L configuration, of the formula:

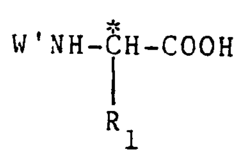 (II)

in which W' represents an N-protecting group such as Boc or Z, and $R_1$ has the meaning indicated above, in a basic medium, in the presence of an activator for the amino acid (II) or of a coupling agent,
(b) then heating the resulting compound of the formula (III):

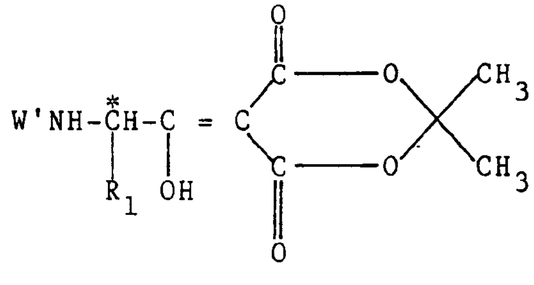

(III)

in which W' and $R_1$ are as defined above, in a solvent at a temperature of between 30°C and 100°C, (c) then reducing the compound (IV-IVa) thus obtained in 2 tautomeric forms in equilibrium, in which the chirality of the carbon atom carrying the substituent $R_1$ is preserved, the said compound being represented by the following structures:

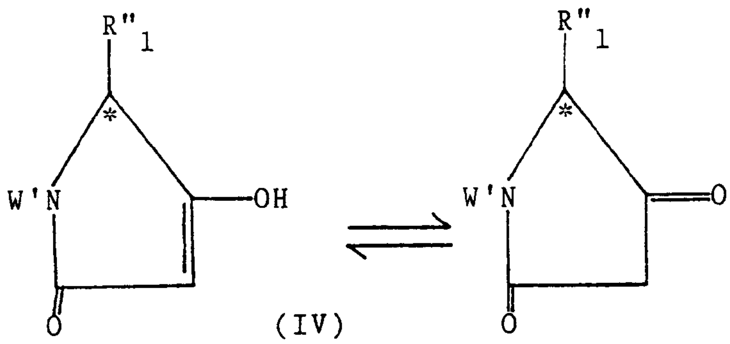

(IV) (IVa)

in which W' and $R_1$ are as defined above, with a metal borohydride in an acid medium, or by catalytic reduction under hydrogen pressure when $R_1$ is not sensitive to hydrogenation
(d) opening the ring of the resulting compound V of the formula:

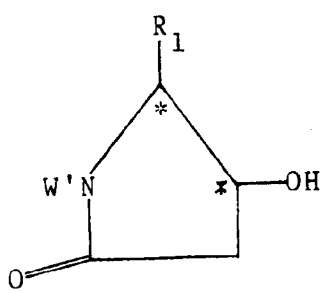

(V)

in which W' and $R_1$ are as defined above, either in an acid medium in the case where W' represents an N-protecting group which is stable in an acid medium, or in a basic medium in the case where W' represents a sterically hindered N-protecting group which is labile in an acid medium, and if necessary, finally deprotecting the nitrogen by removing the N-protecting group from the resulting compound of the formula:

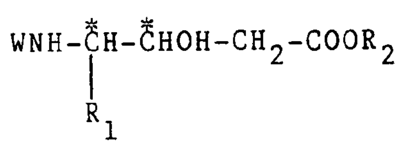

by known methods.

2. Process according to claim 1, characterized in that:
(1) the compound of formula II is Boc-L leucine of formula

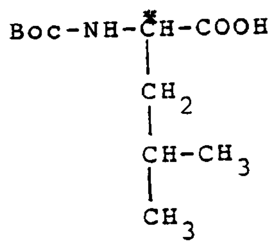

2) and in that step d) is carried out in a basic medium; in that optionally a deprotection of the nitrogen of the compound thus obtained is carried out and in that the (3S,4S)-4-amino-3-hydroxy-6-methyl-heptanoic acid, i.e. the statine, is isolated.

**3.** Process according to claim 1 in which $R_1$ represents a linear or branched alkyl radical having 1 to 6 carbon atoms, a benzyl, a cyclohexylmethyl, a methylthio-lower alkyl, a benzyloxy-lower alkyl, an amino-lower alkyl which is free or substituted on the amine group by an N-protecting group, or an indol-3-ylmethyl.

**4.** Derivatives of optically pure (3R,4R)-4-amino-3-hydroxycarboxylic or (3S,4S)-4-amino-3-hydroxycarboxylic acids of the formula:

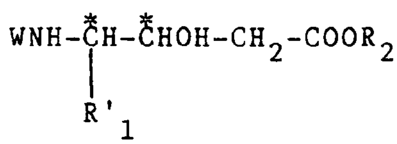

(VII)

in which W and $R_2$ have the same meanings as in claim 1 and $R'_1$ has the same meaning as $R_1$ in claim 1 with the proviso that $R'_1$ is other than:

- ($C_1$-$C_6$) alkyl ;
- Cy-A in which Cy is an aromatic or alicyclic hydrocarbon radical and A is a ($C_1$-$C_5$) alkylene ;
- benzyl which is unsubstituted or substituted by a nitro, omega-aminobutyl which is unsubstituted or substituted on the amine by a protecting group,
- omega-amino-omega carboxybutyl.

**5.** Optically pure compound according to claim 4, in which $R'_1$ represents a methylthio-lower alkyl, a benzyloxy-lower alkyl, or an indol-3-ylmethyl.

**6.** As a synthesis intermediate which can be used in the process according to any one of claims 1 to 3, the optically pure, compounds of the formula:

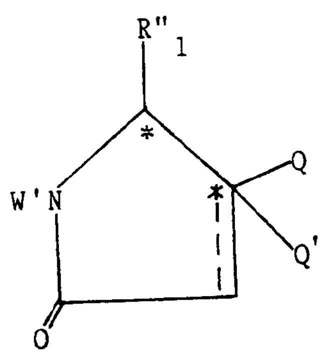

(VI)

in which W' is as defined in claim 1, $R''_1$ has the same meaning as $R_1$ in claim 1 with the restriction that $R''_1$ cannot be 4-aminobutyl, or 4-amino-4-carboxybutyl, Q represents a hydroxyl group, Q' is non-existent or represents a hydrogen atom, or alternatively Q and Q', taken together, represent an oxygen atom, and the broken line represents an additional bond in the case where Q is non-existent, it being understood that, if Q' represents a hydrogen atom, the compound (VI) is in the (S,S) or (R,R) configuration.

7. As a synthesis intermediate which can be used in the process, an optically pure compound according to claim 6 in which R"$_1$ represents a linear or branched alkyl radical having 1 to 6 carbon atoms, a benzyl, a cyclohexylmethyl, a methylthio-lower alkyl, a benzyloxy-lower alkyl or an indol-3-ylmethyl.

8. As synthesis intermediates which can be used in the process according to any one of claims 1 to 3, the optically pure compounds of the formula:

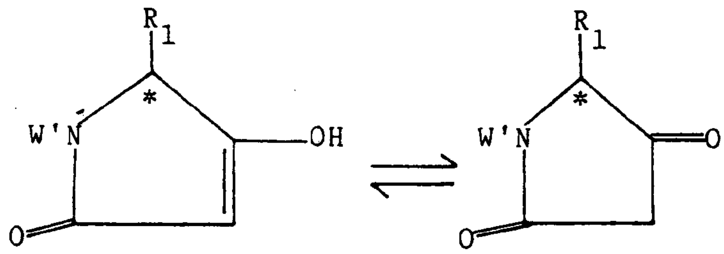

(IV) (IVa)

in which W' has the same meaning as in claim 1 and R"$_1$ has the same meaning as R$_1$ in claim 1 with the restriction that R"$_1$ is different from 4-aminobutyl or 4-amino-4-carboxybutyl.

9. As synthesis intermediates which can be used in the process according to any one of claims 1 to 3, the compounds in the (R,R) or (S,S) configuration, of the formula:

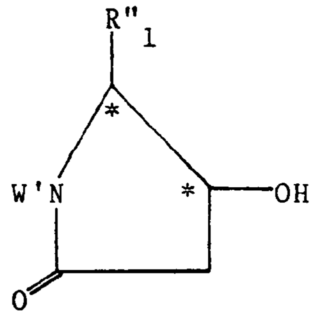

(V)

in which W' has the meaning indicated in claim 1 and R"$_1$ has the same meaning as R$_1$ in claim 1 with the restriction that R"$_1$ is different from 4-aminobutyl or 4-amino-4-carboxybutyl.

10. An optically pure compound of the formula:

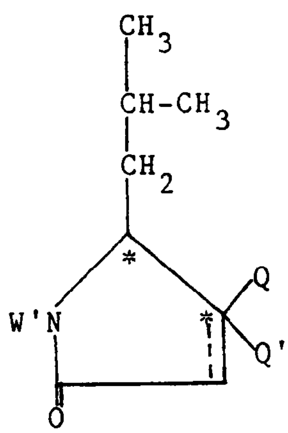

in which W' is as defined in claim 1 and Q and Q' are as defined in claim 6.

**11.** An optically pure compound of the formula:

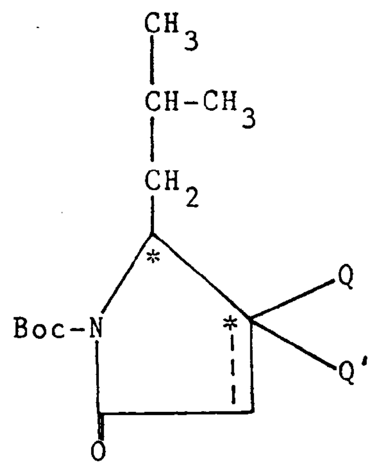

in which Q and Q' are as defined in claim 6.

**12.** A compound, in the (R,R) or (S,S) configuration, of the formula:

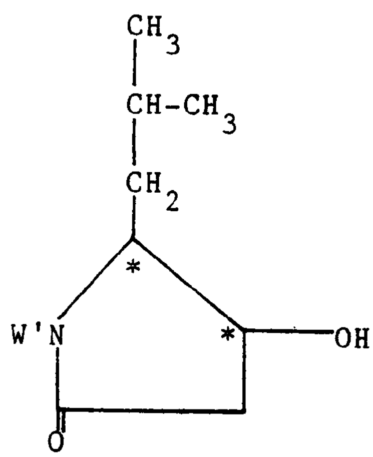

in which W' is as defined in claim 1.

**13.** A compound, in the (R,R) or (S,S) configuration, of the formula:

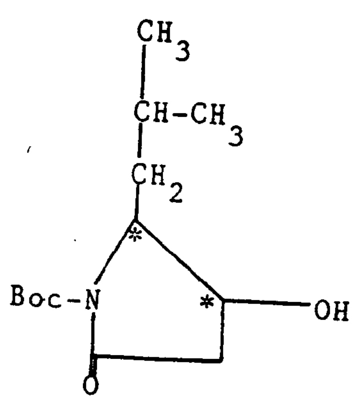

**14.** A compound as claimed in one of claims 4 to 10, in the (S,S) configuration.

## Patentansprüche

**1.** Verfahren zur stereospezifischen Herstellung von optisch reinen (3R,4R)-bzw. (3S,4S)-4-Amino-3-hydroxy-carbonsäurederivaten der Formel

$$WNH - \overset{*}{\underset{\displaystyle R_1}{CH}} - \overset{*}{CHOH} - CH_2 - COOR_2 \qquad (I)$$

worin:

- W Wasserstoff oder eine N-Schutzgruppe ist;
- $R_1$ eine gerade oder verzweigte Alkylgruppe mit 1 bis 6 Kohlenstoffatomen, eine Methyloxyniedrigalkylgruppe, eine Benzyloxyniedrigalkylgruppe, eine Methylthioniedrigalkylgruppe, eine Phenylthioniedrigalkylgruppe, eine Benzylthioniedrigalkylgruppe, eine freie oder durch eine Schutzgruppe an der Aminfunktion substituierte Aminoniedrigalkylgruppe, eine (Niedrigalkyl)-aminoniedrigalkylgruppe, eine (Diniedrigalkyl)-aminoniedrigalkylgruppe, eine Hydroxyniedrigalkylgruppe, eine freie oder veresterte Carboxyniedrigalkylgruppe oder eine freie oder alkylierte Carboxamidoniedrigalkylgruppe, eine auf einmal und an demselben Kohlenstoffatom durch ein Amin und eine freie oder veresterte Carboxygruppe substituierte Niedrigalkylgruppe, eine gerade oder verzweigte Alkenylgruppe mit 2 bis 6 Kohlenstoffatomen, eine Methyloxyniedrigalkenylgruppe, wie Methyloxyvinyl, eine Phenoxyniedrigalkenylgruppe, eine Benzyloxyalkenylgruppe, eine Methylthioniedrigalkenylgruppe, eine Phenylthioniedrigalkenylgruppe, eine Benzylthioniedrigalkenylgruppe, eine freie oder an der Aminfunktion geschützte Aminoniedrigalkenylgruppe, eine (Niedrigalkyl)-aminoniedrigalkenylgruppe, eine (Diniedrigalkyl)-aminoniedrigalkenylgruppe, eine freie oder veresterte Carboxylalkenylgruppe, eine freie oder alkylierte Carboxamidoniedrigalkenylgruppe, eine gerade oder verzweigte Alkynylgruppe mit 2 bis 6 Kohlenstoffatomen oder auch eine der Gruppen der Formeln

  Cy-A-, Cy-O-A' oder $R_3$S-A'-

  darstellt, worin:
  - . Cy für eine aromatische oder alizyklische Kohlenwasserstoffgruppe oder eine heterozyklische Gruppe mit einem Sauerstoff- oder Schwefelatom oder einem oder zwei Stickstoffatom(en) steht, wobei Cy gegebenenfalls durch eine Hydroxy-, Niedrigalkyl-, Niedrigalkoxy-, Trifluormethyl-, Nitro- oder Halogengruppe mono-, di- oder trisubstituiert ist,
  - . A eine einfache Bindung oder eine gerade oder verzweigte Alkylengruppe mit 1 bis 5 Kohlenstoffatomen oder eine gerade oder verzweigte Alkenylengruppe mit 2 bis 5 Kohlenstoffatomen bedeutet,
  - . A' eine gerade oder verzweigte Alkylengruppe mit 1 bis 5 Kohlenstoffatomen oder eine gerade oder verzweigte Alkenylengruppe mit 2 bis 5 Kohlenstoffatomen darstellt;
  - . $R_3$ für eine S-Schutzgruppe steht;
- $R_2$ Wasserstoff, ein Alkali- oder Erdalkalimetall, ein Niedrigalkyl, ein nicht substituiertes oder durch eine Niedrigalkylgruppe, durch ein Halogen oder durch eine Nitrogruppe substituiertes Benzyl darstellt;

dadurch gekennzeichnet, daß

a) die Meldrumsäure mit einer geschützten optisch reinen Aminosäure der D- bzw. L-Konfiguration der Formel

$$W'NH - \overset{*}{\underset{\displaystyle R_1}{CH}} - COOH \qquad (II)$$

worin W' eine N-Schutzgruppe, wie Boc oder Z darstellt, und $R_1$ die weiter oben angegebene Bedeutung hat, in basischem Milieu in Gegenwart eines Mittels zur Aktivierung der Aminosäure (II) oder eines Kopplungsmittels zur Umsetzung gebracht wird;

b) danach die so erhaltene Verbindung der Formel (III)

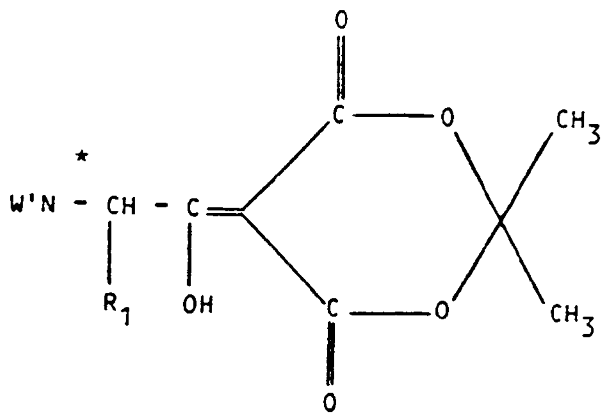

(III)

worin W' und $R_1$ wie oben definiert sind, in einem Lösungsmittel auf eine Temperatur zwischen 30°C und 100°C erhitzt wird;

c) danach die so erhaltene Verbindung (IV-IVa) in zwei Tautomerenformen im Gleichgewicht, in denen die Chiralität des den Substituenten $R_1$ tragenden Kohlenstoffatoms beibehalten ist, dargestellt durch die folgenden Strukturen

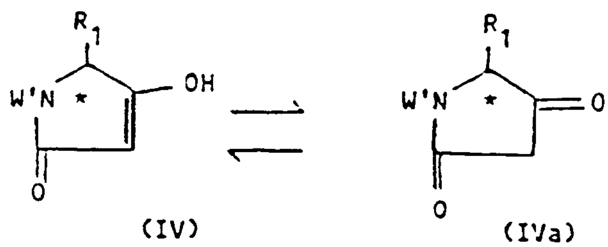

(IV) (IVa)

worin W' und $R_1$ wie oben definiert sind, mit einem metallischen Borhydrid in saurem Milieu oder durch katalytische Reduktion unter Wasserstoffdruck, wenn der Substituent $R_1$ auf die Hydrierung nicht empfindlich ist, reduziert wird;

d) der Ring der so erhaltenen Verbindung (V) der Formel

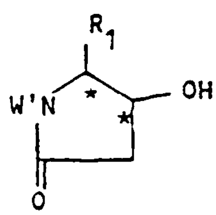

(V)

worin W' und $R_1$ wie oben definiert sind, entweder in saurem Milieu, wenn W' eine in saurem Milieu stabile N-Schutzgruppe darstellt, oder in basischem Milieu, wenn W' eine sterisch raumbeanspruchende und in saurem Milieu labile N-Schutzgruppe ist, geöffnet wird; und notwendigenfalls schließlich eine Entschützung des Stickstoffs durch Entfernen der N-Schutzgruppe aus der so erhaltenen Verbindung der Formel

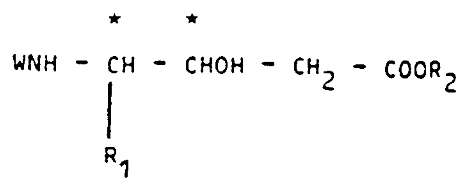

nach bekannten Verfahren erfolgt.

2. Verfahren nach Anspruch 1, dadurch gekennzeichnet, daß
1) die Verbindung der Formel (II) Boc-L-Leucin der Formel

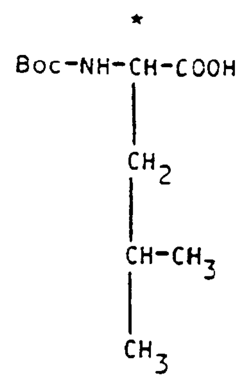

ist;
2) und daß die Stufe d) in basischem Milieu ausgeführt wird; daß gegebenenfalls eine Entschützung des Stickstoffs der so erhaltenen Verbindung erfolgt, und daß die (3S,4S)-4-Amino-3-hydroxy-6-methyl-heptansäure, d.h. Statin, isoliert wird.

3. Verfahren nach Anspruch 1, worin $R_1$ eine gerade oder verzweigte Alkylgruppe mit 1 bis 6 Kohlenstoffatomen, Benzyl, Cyclohexylmethyl, Methylthioniedrigalkyl, Benzyloxyniedrigalkyl, freies oder an der Aminfunktion durch eine N-Schutzgruppe substituiertes Aminoniedrigalkyl, 3-Indolylmethyl darstellt.

4. Optische reine (3R,4R)- bzw. (3S,4S)-4-Amino-3-hydroxy-carbonsäurederivate der Formel

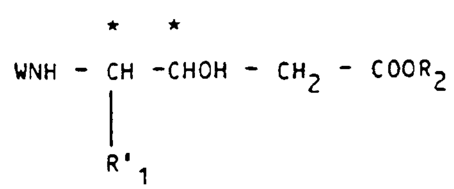

(VII)

worin W und $R_2$ dieselben Bedeutungen wie in Anspruch 1 haben und $R'_1$ dieselbe Bedeutung wie $R_1$ hat, mit der Maßgabe, daß $R'_1$ nicht:
- $C_1$-$C_6$-Alkyl;
- Cy-A, worin Cy eine aromatische oder alizyklische Kohlenwasserstoffgruppe und A ein $C_1$-$C_5$-Alkylen darstellt;
- durch eine Nitrogruppe substituiertes Benzyl oder am Amin gegebenenfalls durch eine Schutzgruppe substituiertes omega-Aminobutyl;
- omega-Amino-omega-Carboxybutyl ist.

5. Optisch reine Verbindungen nach Anspruch 4, dadurch gekennzeichnet, daß $R'_1$ eine Methylthioniedrigalkyl-, eine Benzyloxyniedrigalkyl-oder eine 3-Indolylmethylgruppe darstellt.

6. Als Synthesezwischenprodukte beim Verfahren nach einem der Ansprüche 1 bis 3 nützliche optisch reine Verbindungen der Formel

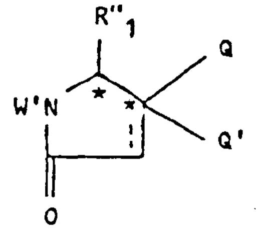

(VI)

worin W' wie in Anspruch 1 definiert ist, R"$_1$ dieselbe Bedeutung wie $R_1$ des Anspruchs 1 hat, mit der Einschränkung, daß R"$_1$ nicht 4-Aminobutyl oder 4-Amino-4-carboxybutyl ist, Q eine Hydroxylgruppe darstellt, Q' nicht existiert oder ein Wasserstoffatom darstellt, oder aber Q und Q' zusammen ein Sauerstoffatom bedeuten und die punktierte Linie, wenn Q' nicht existiert, eine zusätzliche Bindung darstellt, mit der Maßgabe, daß die Verbindung (VI), wenn Q' ein Wasserstoffatom darstellt, (S,S)- oder (R,R)-Konfiguration hat.

7. Als Synthesezwischenprodukt beim Verfahren nützliche optisch reine Verbindung nach Anspruch 6, worin R"$_1$ eine gerade oder verzweigte Alkylgruppe mit 1 bis 6 Kohlenstoffatomen, Benzyl, Cyclohexylmethyl, Methylthioniedrigalkyl, Benzyloxyniedrigalkyl, 3-Indolylmethyl darstellt.

8. Als Synthesezwischenprodukte beim Verfahren nach einem der Ansprüche 1 bis 3 nützliche optisch reine Verbindungen der Formel

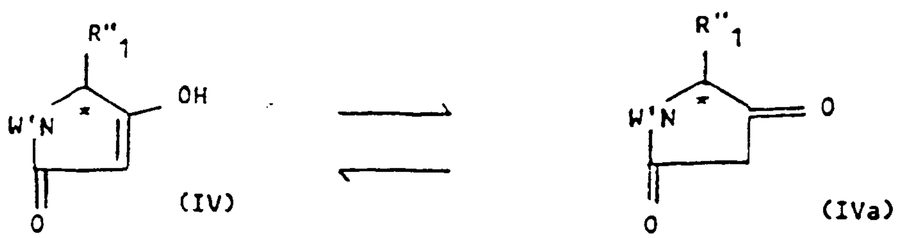

(IV) (IVa)

worin W' dieselbe Bedeutung wie in Anspruch 1 hat und R"$_1$ dieselbe Bedeutung wie $R_1$ des Anspruchs 1 hat, mit der Einschränkung, daß R"$_1$ nicht 4-Aminobutyl oder 4-Amino-4-carboxybutyl ist.

9. Als Synthesezwischenprodukte beim Verfahren nach einem der Ansprüche 1 bis 3 nützliche Verbindungen mit (R,R)- oder (S,S) -Konfiguration der Formel

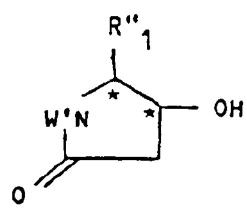

(V)

worin W' die in Anspruch 1 angegebene Bedeutung hat und R"$_1$ dieselbe Bedeutung wie $R_1$ des Anspruchs 1 hat, mit der Einschränkung, daß R"$_1$ nicht 4-Aminobutyl oder 4-Amino-4-carboxybutyl ist.

10. Optisch reine Verbindung der Formel

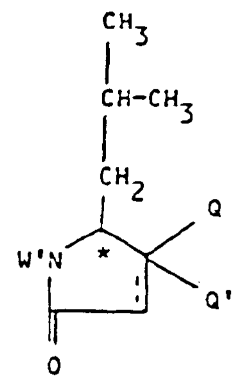

worin W' wie in Anspruch 1 definiert ist und Q und Q' wie in Anspruch 6 definiert sind.

**11.** Optisch reine Verbindung der Formel

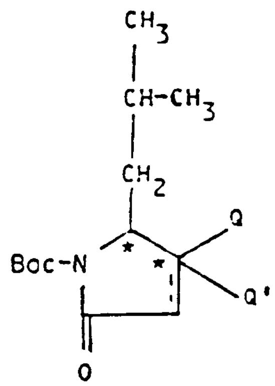

worin Q und Q' wie in Anspruch 6 definiert sind.

**12.** Verbindung mit (R,R)- oder (S,S)-Konfiguration der Formel

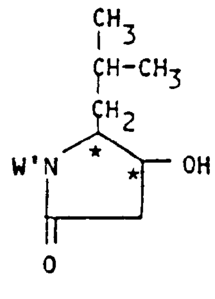

worin W' wie in Anspruch 1 definiert ist.

**13.** Verbindung mit (R,R)- oder (S,S)-Konfiguration der Formel

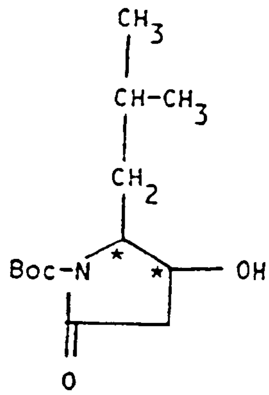

**14.** Verbindung nach einem der Ansprüche 4 bis 10 mit (S,S)-Konfiguration.